# EUROPÄISCHE PATENTSCHRIFT

(11) **EP 1 824 844 B1**
(45) Veröffentlichungstag und Bekanntmachung des Hinweises auf die Patenterteilung: **05.11.2008**
(21) Anmeldenummer: 05815232.3
(22) Anmeldetag: 28.11.2005
(51) Int. Cl.: C07D 403/12, C07D 401/14, C07D 413/12, C07D 413/14, A61K 31/5377, A61P 7/02

(54) **PYRAZINDICARBONSÄUREAMIDE UND IHRE VERWENDUNG**
PYRAZINE DICARBOXAMIDES AND THE USE THEREOF
AMIDES D'ACIDE DICARBOXYLIQUE DE PYRAZINE ET LEUR UTILISATION

(30) Priorität: 09.12.2004 DE 102004059219
(43) Veröffentlichungstag der Anmeldung: 29.08.2007
(73) Patentinhaber: Bayer HealthCare AG, 51368 Leverkusen (DE)
(72) Erfinder: RÖHRIG, Susanne, 45276 Essen (DE); JESKE, Mario, 42699 Solingen (DE); AKBABA, Metin, 40880 Ratingen (DE); ROSENTRETER, Ulrich, 31619 Binnen (DE); BOYER, Stephen, Bethany, Connecticut 06524 (US); FISCHER, Karin, 42699 Solingen (DE); POHLMANN, Jens, CH-4058 Basel (CH); TUCH, Arounarith, F-69266 Lyon (FR); PERZBORN, Elisabeth, 42327 Wuppertal (DE); GERDES, Christoph, 51373 Leverkusen (DE); SCHLEMMER, Karl-Heinz, 42113 Wuppertal (DE); BURKHARDT, Nils, 42553 Velbert (DE); ALLERHEILIGEN, Swen, 45259 Essen (DE); NELL, Peter, 42115 Wuppertal (DE); ARNDT, Sabine, 44227 Dortmund (DE); LOBELL, Mario, 42113 Wuppertal (DE)
(86) Internationale Anmeldenummer: PCT/EP2005/012681
(87) Internationale Veröffentlichungsnummer: WO 2006/061116

(56) Entgegenhaltungen:
- WO-A-02/079145
- WO-A-03/026652

## Beschreibung

Die vorliegende Anmeldung betrifft neue Pyrazindicarbonsäureamide, Verfahren zu ihrer Herstellung, ihre Verwendung zur Verhinderung der Blutkoagulation in vitro sowie ihre Verwendung zur Herstellung von Arzneimitteln zur Behandlung und/oder Prophylaxe von Krankheiten, insbesondere von thromboembolischen Erkrankungen.

Die Blutgerinnung ist ein Schutzmechanismus des Organismus, mit dessen Hilfe Defekte in der Gefäßwand rasch und zuverlässig "abgedichtet" werden können. So kann ein Blutverlust vermieden bzw. minimiert werden. Die Blutstillung nach Gefäßverletzung erfolgt im wesentlichen durch das Gerinnungssystem, bei dem eine enzymatische Kaskade komplexer Reaktionen von Plasmaproteinen ausgelöst wird. Hierbei sind zahlreiche Blutgerinnungsfaktoren beteiligt, von denen jeder, sobald aktiviert, die jeweils nächste inaktive Vorstufe in ihre aktive Form überführt. Am Ende der Kaskade steht die Umwandlung des löslichen Fibrinogens in das unlösliche Fibrin, so dass es zu einem Blutgerinnsel kommt. Traditionell unterscheidet man bei der Blutgerinnung zwischen dem intrinsischen und dem extrinsischen System, die in einem abschließenden gemeinsamen Reaktionsweg münden. Hierbei kommt dem Faktor Xa, der aus dem Proenzym Faktor X gebildet wird, eine Schlüsselrolle zu, da er beide Gerinnungswege verbindet. Die aktivierte Serinprotease Xa spaltet Prothrombin zu Thrombin. Das entstandene Thrombin wiederum spaltet seinerseits Fibrinogen zu Fibrin. Durch anschließende Quervernetzung der Fibrin-Monomere kommt es zur Bildung von Blutgerinnseln und damit zur Blutstillung. Darüber hinaus ist Thrombin ein potenter Auslöser der Thrombozytenaggregation, die ebenfalls einen erheblichen Beitrag bei der Hämostase leistet.

Die Hämostase unterliegt einem komplexen Regulationsmechanismus. Eine unkontrollierte Aktivierung des Gerinnungssystems oder eine defekte Hemmung der Aktivierungsprozesse kann die Bildung von lokalen Thrombosen oder Embolien in Gefäßen (Arterien, Venen, Lymphgefäßen) oder Herzhöhlen bewirken. Dies kann zu schwerwiegenden thromboembolischen Erkrankungen führen. Darüber hinaus kann eine Hyperkoagulabilität - systemisch - bei einer Verbrauchskoagulopathie zur disseminierten intravasalen Gerinnung führen. Thromboembolische Komplikationen treten ferner auf bei mikroangiopathischen hämolytischen Anämien, extrakorporalen Blutkreisläufen, wie Hämodialyse, sowie Herzklappenprothesen.

Thromboembolische Erkrankungen sind die häufigste Ursache von Morbidität und Mortalität in den meisten industrialisierten Ländern [Heart Disease: A Textbook of Cardiovascular Medicine, Eugene Braunwald, 5. Auflage, 1997, W.B. Saunders Company, Philadelphia].

Die aus dem Stand der Technik bekannten Antikoagulantien, d.h. Stoffe zur Hemmung oder Verhinderung der Blutgerinnung, weisen verschiedene, oftmals gravierende Nachteile auf. Eine effiziente Behandlungsmethode bzw. Prophylaxe von thromboembolischen Erkrankungen erweist sich in der Praxis deshalb als sehr schwierig und unbefriedigend.

Für die Therapie und Prophylaxe von thromboembolischen Erkrankungen findet zum einen Heparin Verwendung, das parenteral oder subkutan appliziert wird. Aufgrund günstigerer pharmakokinetischer Eigenschaften wird zwar heutzutage zunehmend niedermolekulares Heparin bevorzugt; allerdings können auch hierdurch die im folgenden geschilderten bekannten Nachteile nicht vermieden werden, die bei der Therapierung mit Heparin bestehen. So ist Heparin oral unwirksam und besitzt nur eine vergleichsweise geringe Halbwertszeit. Da Heparin gleichzeitig mehrere Faktoren der Blutgerinnungskaskade hemmt, kommt es zu einer unselektiven Wirkung. Darüber hinaus besteht ein hohes Blutungsrisiko, insbesondere können Hirnblutungen und Blutungen im Gastrointestinaltrakt auftreten, und es kann zu Thrombopenie, Alopecia medicomentosa oder Osteoporose kommen [Pschyrembel, Klinisches Wörterbuch, 257. Auflage, 1994, Walter de Gruyter Verlag, Seite 610, Stichwort "Heparin"; Römpp Lexikon Chemie, Version 1.5, 1998, Georg Thieme Verlag Stuttgart, Stichwort "Heparin"].

Eine zweite Klasse von Antikoagulantien stellen die Vitamin K-Antagonisten dar. Hierzu gehören beispielsweise 1,3-Indandione, vor allem aber Verbindungen wie Warfarin, Phenprocoumon, Dicumarol und andere Cumarin-Derivate, die unselektiv die Synthese verschiedener Produkte bestimmter Vitamin K-abhängiger Gerinnungsfaktoren in der Leber hemmen. Durch den Wirkmechanismus bedingt, setzt die Wirkung aber nur sehr langsam ein (Latenzzeit bis zum Wirkeintritt 36 bis 48 Stunden). Die Verbindungen können zwar oral appliziert werden, aufgrund des hohen Blutungsrisikos und des engen therapeutischen Indexes ist aber eine aufwendige individuelle Einstellung und Beobachtung des Patienten notwendig [J. Hirsh, J. Dalen, D.R. Anderson et al., "Oral anticoagulants: Mechanism of action, clinical effectiveness, and optimal therapeutic range" Chest 2001, 119, 8S-21S; J. Ansell, J. Hirsh, J. Dalen et al., "Managing oral anticoagulant therapy" Chest 2001, 119, 22S-38S; P.S. Wells, A.M. Holbrook, N.R. Crowther et al., "Interactions of warfarin with drugs and food" Ann. Intern. Med. 1994, 121, 676-683].

In jüngster Zeit ist ein neuer Therapieansatz für die Behandlung und Prophylaxe von thromboembolischen Erkrankungen beschrieben worden. Ziel dieses neuen Therapieansatzes ist die Inhibierung von Faktor Xa. Entsprechend der zentralen Rolle, die Faktor Xa in der Blutgerinnungskaskade spielt, stellt Faktor Xa eines der wichtigsten Targets für antikoagulatorische Wirkstoffe dar [J. Hauptmann, J. Stürzebecher, Thrombosis Research 1999, 93, 203; S.A.V. Raghavan, M. Dikshit, "Recent advances in the status and targets of antithrombotic agents" Drugs Fut. 2002, 27, 669-683; H.A. Wieland, V. Laux, D. Kozian, M. Lorenz, "Approaches in anticoagulation: Rationales for target positioning" Curr. Opin. Investig. Drugs 2003, 4, 264-271; U.J. Ries, W. Wienen, "Serine proteases as targets for antithrombotic therapy" Drugs Fut. 2003, 28, 355-370; L.-A. Linkins, J.I. Weitz, "New anticoagulant therapy" Annu. Rev. Med. 2005, 56, 63-77 (online-Publikation August 2004)].

Dabei ist gezeigt worden, dass verschiedene, sowohl peptidische wie nicht-peptidische Verbindungen in Tiermodellen als Faktor Xa-Inhibitoren wirksam sind. Eine große Anzahl von direkten Faktor Xa-Inhibitoren ist bislang bekannt [J.M. Walenga, W.P. Jeske, D. Hoppensteadt, J. Fareed, "Factor Xa Inhibitors: Today and beyond" Curr. Opin. Investig. Drugs 2003, 4, 272-281; J. Ruef, H.A. Katus, "New antithrombotic drugs on the horizon" Expert Opin. Investig. Drugs 2003, 12, 781-797; M.L. Quan, J.M. Smallheer, "The race to an orally active Factor Xa inhibitor: Recent advances" Curr. Opin. Drug Discovery & Development 2004, 7, 460-469]. Weiterhin sind nicht-peptidische, niedermolekulare Faktor Xa-Inhibitoren beispielsweise auch in WO 03/026652, WO 02/079145, WO 01/019788 und WO 01/064642 beschrieben.

Die Aufgabe der vorliegenden Erfindung liegt in der Bereitstellung neuer Substanzen zur Bekämpfung von Erkrankungen, insbesondere von thromboembolischen Erkrankungen.

Gegenstand der vorliegenden Erfindung sind Verbindungen der allgemeinen Formel (I) 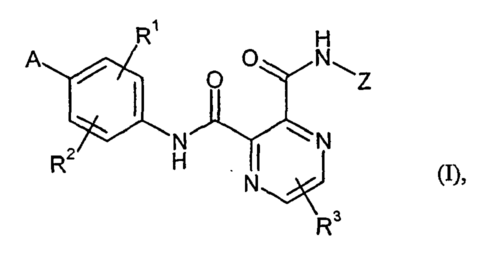 in welcher
- A: für eine Gruppe der Formel 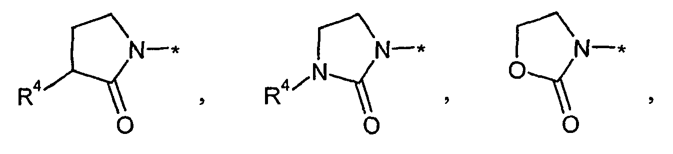 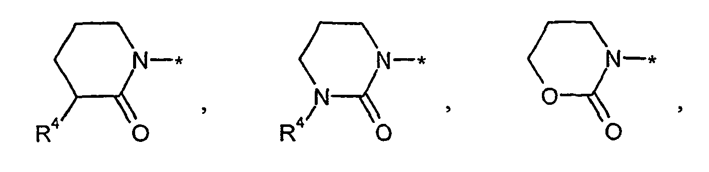 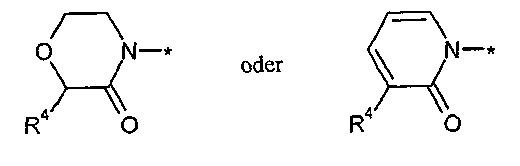 steht, worin
- R⁴: Wasserstoff, (C₁-C₆)-Alkyl, Hydroxy, (C₁-C₆)-Alkoxy, Amino, Mono- oder Di-(C₁-C₆)-alkylamino, (C₃-C₇)-Cycloalkylamino, (C₁-C₆)-Alkanoylamino oder (C₁-C₆)-Alkoxycarbonylamino bedeutet, wobei
(C₁-C₆)-Alkyl, (C₁-C₆)-Alkoxy, Mono- und Di-(C₁-C₆)-alkylamino ihrerseits jeweils durch Hydroxy, (C₁-C₄)-Alkoxy, Amino, Mono- oder Di-(C₁-C₄)-alkylamino, (C₃-C₇)-Cycloakylamino oder einen 4- bis 7-gliedrigen gesättigten, über ein N-Atom gebundenen Heterocyclus, der ein Ringglied aus der Reihe N-R⁵ oder O enthalten kann, worin
- R⁵: Wasserstoff oder (C₁-C₄)-Alkyl bedeutet,
substituiert sein können,
und * die Verknüpfungsstelle mit dem Phenylring bedeutet,
- Z: für Phenyl, Pyridyl, Pyrimidinyl, Pyrazinyl oder Thienyl steht, die jeweils ein- oder zweifach, gleich oder verschieden, durch Substituenten ausgewählt aus der Gruppe von Fluor, Chlor, Cyano, (C₁-C₄)-Alkyl, welches seinerseits durch Amino substituiert sein kann, Ethinyl und Amino substituiert sein können,
- R¹ und R²: gleich oder verschieden sind und unabhängig voneinander für Wasserstoff, Fluor, Chlor, Cyano, (C₁-C₃)-Alkyl, Cyclopropyl, Trifluormethyl, Hydroxy, (C₁-C₃)-Alkoxy, Trifluormethoxy oder Amino stehen, wobei (C₁-C₃)-Alkyl und (C₁-C₃)-Alkoxy ihrerseits durch Hydroxy oder Amino substituiert sein können,
und
- R³: für Wasserstoff oder (C₁-C₆)-Alkyl, welches durch Hydroxy, (C₁-C₄)-Alkoxy, Amino oder Mono- oder Di-(C₁-C₄)-alkylamino substituiert sein kann, steht,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der *Salze handelt*.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Offenbarung auch Prodrugs der erfindungsgemäßen Verbindungen. Der Begriff "Prodrugs" umfasst Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
(C₁-C₆ )-Alkyl, (C₁-C₄)-Alkyl und (C₁-C₃)-Alkyl stehen im Rahmen der Erfindung für einen geradkettigen oder verzweigten Alkylrest mit 1 bis 6, 1 bis 4 bzw. 1 bis 3 Kohlenstoffatomen. Bevorzugt ist ein geradkettiger oder verzweigter Alkylrest mit 1 bis 4 bzw. 1 bis 3 Kohlenstoffatomen. Besonders bevorzugt ist ein geradkettiger oder verzweigter Alkylrest mit 1 bis 3 Kohlenstoffatomen. Beispielhaft und vorzugsweise seien genannt: Methyl, Ethyl, n-Propyl, Isopropyl, n-Butyl, isoButyl, sec.-Butyl, tert.-Butyl, 1-Ethylpropyl, n-Pentyl und n-Hexyl.
(C₃-C₇)-Cycloalkyl steht im Rahmen der Erfindung für eine monocyclische Cycloalkylgruppe mit 3 bis 7 Kohlenstoffatomen. Bevorzugt ist ein Cycloalkylrest mit 3 bis 6 Kohlenstoffatomen. Beispielhaft und vorzugsweise seien genannt: Cyclopropyl, Cyclobutyl, Cyclopentyl, Cyclohexyl und Cycloheptyl.
(C₁-C₆)-Alkoxy, (C₁₋C₄)-Alkoxy und (C₁-C₃)-Alkoxy stehen im Rahmen der Erfindung für einen geradkettigen oder verzweigten Alkoxyrest mit 1 bis 6, 1 bis 4 bzw. 1 bis 3 Kohlenstoffatomen.

Bevorzugt ist ein geradkettiger oder verzweigter Alkoxyrest mit 1 bis 4 bzw. 1 bis 3 Kohlenstoffatomen. Besonders bevorzugt ist ein geradkettiger oder verzweigter Alkoxyrest mit 1 bis 3 Kohlenstoffatomen. Beispielhaft und vorzugsweise seien genannt: Methoxy, Ethoxy, n-Propoxy, Isopropoxy, n-Butoxy und tert.-Butoxy.

C₁-C₂)-Alkanoyl [(C₁-C₆)-Acyl] steht im Rahmen der Erfindung für einen geradkettigen oder verzweigten Alkylrest mit 1 bis 6 Kohlenstoffatomen, der in der 1-Position ein doppelt gebundenes Sauerstoffatom trägt und über die 1-Position verknüpft ist. Bevorzugt ist ein geradkettiger oder verzweigter Alkanoylrest mit 1 bis 4 Kohlenstoffatomen. Beispielhaft und vorzugsweise seien genannt: Formyl, Acetyl, Propionyl, n-Butyryl, iso-Butyryl und Pivaloyl:
(C₁-C₆)-Alkoxycarbonyl steht im Rahmen der Erfindung für einen geradkettigen oder verzweigten Alkoxyrest mit 1 bis 6 Kohlenstoffatomen, der über eine Carbonylgruppe verknüpft ist. Bevorzugt ist ein geradkettiger oder verzweigter Alkoxycarbonylrest mit 1 bis 4 Kohlenstoffatomen in der Alkoxy-Gruppe. Beispielhaft und vorzugsweise seien genannt: Methoxycarbonyl, Ethoxycarbonyl, n-Propoxycarbonyl, Isopropoxycarbonyl und tert.-Butoxycarbonyl.

Mono-(C₁-C₆)-Alkylamino und Mono-(C₁-C₄-Alkylamino stehen im Rahmen der Erfindung für *eine Amino-Gruppe mit einem geradkettigen oder verzweigten Alkylsubstituenten, der 1 bis 6 bzw*. 1 bis 4 Kohlenstoffatome aufweist. Bevorzugt ist ein geradkettiger oder verzweigter Monoalkylamino-Rest mit 1 bis 4 Kohlenstoffatomen. Beispielhaft und vorzugsweise seien genannt: Methylamino, Ethylamino, n-Propylamino, Isopropylamino und tert.-Butylamino.

Di-(C₁-C₆)-Alkylamino und Di-(C₁-C₄)-Alkylamino stehen im Rahmen der Erfindung für eine Amino-Gruppe mit zwei gleichen oder verschiedenen geradkettigen oder verzweigten Alkylsubstituenten, die jeweils 1 bis 6 bzw. 1 bis 4 Kohlenstoffatome aufweisen. Bevorzugt sind geradkettige oder verzweigte Dialkylamino-Reste mit jeweils 1 bis 4 Kohlenstoffatomen. Beispielhaft und vorzugsweise seien genannt: *N,N*-Dimethylamino, *N,N-*Diethylamino, *N*-Ethyl-*N*-methylamino, *N*-Methyl-*N*-n-propylamino, *N*-Isopropyl-*N*-n-propylamino, *N*-tert.-Butyl-*N*-methylamino, *N*-Ethyl-*N*-n-pentylamino und *N*-n-Hexyl-*N-*methylamino.

(C₃-C₇)-Cycloalkylamino steht im Rahmen der Erfindung für eine Amino-Gruppe mit einem Cycloalkyl-Substituenten, der 3 bis 7 Kohlenstoffatome aufweist. Bevorzugt ist ein Cycloalkylamino-Rest mit 3 bis 6 Kohlenstoffatomen. Beispielhaft und vorzugsweise seien genannt: Cyclopropylamino, Cyclobutylamino, Cyclopentylamino, Cyclohexylamino und Cycloheptylamino.

(C₁-C₆)-Alkanoylamino steht im Rahmen der Erfindung für eine Amino-Gruppe mit einem geradkettigen oder verzweigten Alkanoyl-Substituenten, der 1 bis 6 Kohlenstoffatome aufweist und über die Carbonylgruppe verknüpft ist. Bevorzugt ist ein Alkanoylamino-Rest mit 1 bis 4 Kohlenstoffatomen. Beispielhaft und vorzugsweise seien genannt: Formamido, Acetamido, Propionamido, n-Butyramido und Pivaloylamido.

(C₁-C₆)-Alkoxycarbonylamino steht im Rahmen der Erfindung für eine Amino-Gruppe mit einem geradkettigen oder verzweigten. Alkoxycarbonyl-Substituenten, der im Alkoxyrest 1 bis 6 Kohlenstoffatome aufweist und über die Carbonylgruppe verknüpft ist. Bevorzugt ist ein Alkoxycarbonylamino-Rest mit 1 bis 4 Kohlenstoffatomen in der Alkoxy-Gruppe. Beispielhaft und vorzugsweise seien genannt: Methoxycarbonylamino, Ethoxycarbonylamino, n-Propoxycarbonylamino und tert.-Butoxycarbonylamino.

Ein 4- bis 7-gliedriger Heterocyclus steht im Rahmen der Erfindung für einen gesättigten Heterocyclus mit 4 bis 7 Ringatomen, der ein Ring-Stickstoffatom enthält, über dieses verknüpft ist und ein weiteres Heteroatom aus der Reihe N oder O enthalten kann. Bevorzugt ist ein 5- oder 6-gliedriger gesättigter, N-verknüpfter Heterocyclus, der ein weiteres Heteroatom aus der Reihe N oder O enthalten kann. Beispielhaft seien genannt: Pyrrolidinyl, Oxazolidinyl, Imidazolidinyl, Piperidinyl, Piperazinyl, Morpholinyl, Azepinyl und 1,4-Diazepinyl. Besonders bevorzugt sind Pyrrolidinyl, Piperidinyl, Piperazinyl und Morpholinyl.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem Substituenten.

Bevorzugt sind Verbindungen der Formel (I), in welcher
- A: für eine Gruppe der Formel 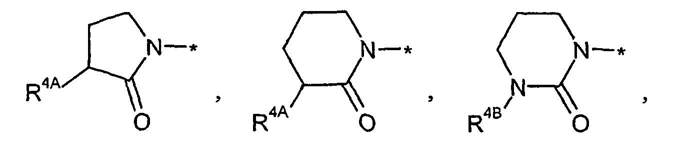 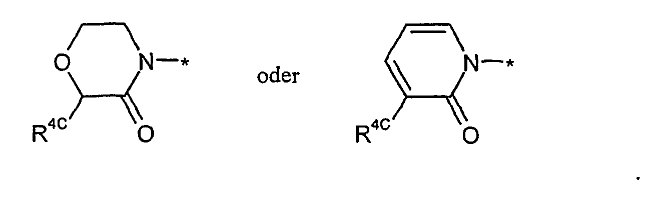 steht, worin
R^{4A} Wasserstoff, Hydroxy, Methoxy oder Amino,
R^{4B} Methyl oder Ethyl, welche jeweils durch Hydroxy, Amino, Pyrrolidino oder Cyclopropylamino substituiert sein können, oder Amino,
R^{4C} Wasserstoff, Methyl oder Ethyl, wobei Methyl oder Ethyl jeweils durch Hydroxy, Amino, Pyrrolidino oder Cyclopropylamino substituiert sein können,
und
* die Verknüpfungsstelle mit dem Phenylring bedeuten,
- Z: für eine Gruppe der Formel 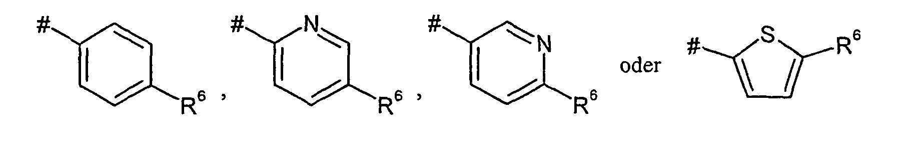 steht, worin
R⁶ Fluor, Chlor, Methyl, Cyano oder Ethinyl
und
# die Verknüpfungsstelle mit dem Stickstoffatom bedeutet,
- R¹: für Wasserstoff,
- R²: für Wasserstoff, Fluor oder Methyl,
und
- R³: für Wasserstoff stehen,
sowie ihre Salze, Solvate und Solvate der Salze.

Besonders bevorzugt sind Verbindungen der Formel (I), in welcher
- A: für eine heterocyclische Gruppe der Formel 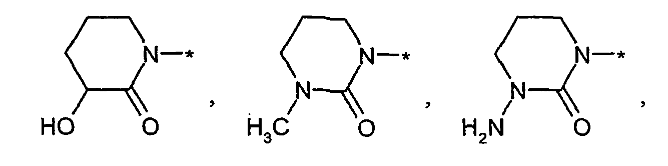 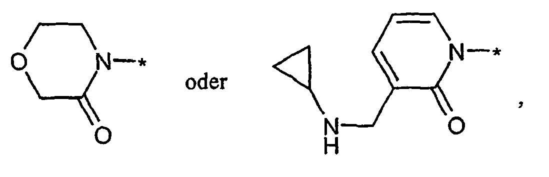 worin * die Verknüpfungsstelle mit dem Phenylring bedeutet,
- Z: für eine Gruppe der Formel 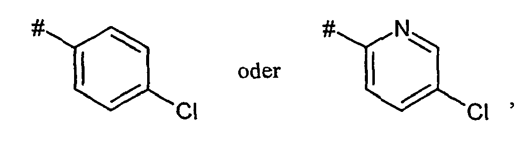 worin # die Verknüpfungsstelle mit dem Stickstoffatom bedeutet,
- R¹: für Wasserstoff,
- R²: für Wasserstoff, Fluor oder Methyl,
und
- R³: für Wasserstoff stehen,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen, dadurch gekennzeichnet, dass man entweder
[A] Verbindungen der Formel (II) 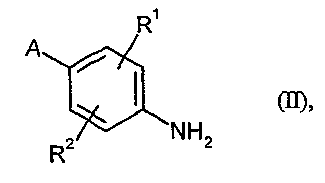 in welcher A, R¹ und R² die oben angegebenen Bedeutungen haben,
   zunächst in einem inerten Lösungsmittel in Gegenwart einer Base, wie beispielsweise Triethylamin, und eines wasserentziehenden Mittels, wie beispielsweise Pivaloylchlorid, mit einer Verbindung der Formel (III) 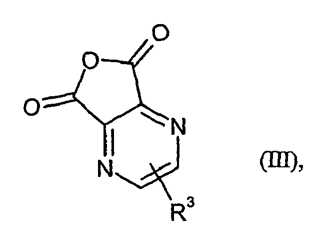 in welcher R³ die oben angegebene Bedeutung hat,
   zu Verbindungen der Formel (IV) 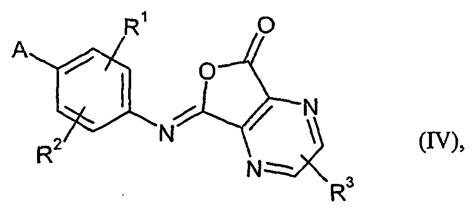 in welcher A, R¹, R² und R³ die oben angegebenen Bedeutungen haben,
   umsetzt und diese dann in einem inerten Lösungsmittel in Gegenwart einer Säure mit einer Verbindung der Formel (V)

   H₂N—Z (V),

   in welcher Z die oben angegebene Bedeutung hat,
   in Verbindungen der Formel (I) überführt
   oder
[B] Verbindungen der Formel (V) zunächst in einem inerten Lösungsmittel gegebenenfalls in Gegenwart einer Base mit einer Verbindung der Formel (III) zu Verbindungen der Formel (VI) 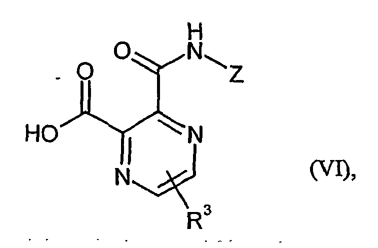 in welcher R³ und Z die oben angegebenen Bedeutungen haben,
   umsetzt und diese dann in einem inerten Lösungsmittel nach Aktivierung der Carbonsäure-Funktion mit einer Verbindung der Formel (II) in Verbindungen der Formel (I) überführt,
und die Verbindungen der Formel (I) gegebenenfalls mit den entsprechenden (i) Lösungsmitteln und/oder (ii) Basen oder Säuren zu ihren Solvaten, Salzen und/oder Solvaten der Salze umsetzt.

Die erfindungsgemäßen Verbindungen können gegebenenfalls auch durch *weitere* Umwandlungen von funktionellen Gruppen einzelner Substituenten, insbesondere den unter R³ und R⁴ aufgeführten Substituenten, ausgehend von den nach obigem Verfahren erhaltenen Verbindungen der Formel (I) hergestellt werden. Diese Umwandlungen werden nach üblichen Methoden durchgeführt und umfassen beispielsweise Reaktionen wie Alkylierung, Aminierung, Acylierung, Veresterung, Esterspaltung, Amid-Bildung, Oxidation oder Reduktion sowie die Einführung und Entfernung von Schutzgruppen.

Inerte Lösungsmittel für die Verfahrensschritte (II) + (III) → (IV) und (IV) + (V) → (I) sind beispielsweise Halogenkohlenwasserstoffe wie Dichlormethan, Trichlormethan, Tetrachlormethan, 1,2-Dichlorethan, Trichlorethylen oder Chlorbenzol, oder Lösungsmittel wie Dimethylsulfoxid, Dimethylformamid, *N,N'*-Dimethylpropylenharnstoff (DMPU), *N*-Methylpyrrolidon (NMP) oder Acetonitril. Ebenso ist es möglich, Gemische der genannten Lösungsmittel zu verwenden. Bevorzugt ist Dimethylformamid.

Als Base für den Verfahrensschritt (II) + (III) → (IV) und gegebenenfalls auch für den Verfahrensschritt (V) + (III) → (VI) eignen sich die üblichen organischen Amin-Basen. Hierzu gehören insbesondere Triethylamin, *N-*Methylmorpholin, *N*-Methylpiperidin, *N,N*-Diisopropylethylamin, Pyridin, 1,5-Diazabicyclo[4.3.0]non-5-en (DBN), 1,4-Diazabicyclo[2.2.2]octan (DABCO^{®}) oder 1,8-Diazabicyclo[5.4.0]undec-7-en (DBU). Bevorzugt ist Triethylamin.

Als wasserentziehendes Mittel im Verfahrensschritt (II) + (III) → (IV) eignen sich beispielsweise organische Carbonsäurechloride wie Acetylchlorid oder Pivaloylchlorid, organische Sulfonsäurechloride wie Methansulfonsäurechlorid, Chlorameisensäureester wie Methylchlorformiat oder Isobutylchlorformiat, oder anorganische Säurechloride oder Anhydride wie Phosphoroxychlorid, Phosphorpentachlorid, Phosphortrichlorid, Phosphorpentoxid oder Thionylchlorid. Bevorzugt wird Pivaloylchlorid verwendet.

Als Säure für den Verfahrensschritt (IV) + (V) → (I) eignen sich beispielsweise organische Carbonsäuren oder Sulfonsäuren wie Essigsäure, Trifluoressigsäure, Methansulfonsäure oder Trifluormethansulfonsäure, oder anorganische Säuren wie Chlorwasserstoff, Bromwasserstoff, Schwefelsäure oder Phosphorsäure. Bevorzugt wird Trifluoressigsäure verwendet.

Inerte Lösungsmittel für den Verfahrensschritt (V) + (III) → (VI) sind beispielsweise Halogenkohlenwasserstoffe wie Dichlormethan, Trichlormethan, Tetrachlormethan, Trichlorethan, Tetrachlorethan, 1,2-Dichlorethan oder Trichlorethylen, Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Ethylacetat, Aceton, Dimethylformamid, Dimethylsulfoxid, *N,N'*-Dimethylpropylenharnstoff (DMPU), *N-*Methylpyrrolidon (NMP) oder Acetonitril. Ebenso ist es möglich, Gemische der genannten Lösungsmittel einzusetzen. Bevorzugt ist Tetrahydrofuran oder Dimethylformamid.

Die Verfahrensschritte (II) + (III) → (IV) und (V) + (III) → (VI) werden im Allgemeinen in einem Temperaturbereich von -20°C bis +60°C, bevorzugt von 0°C bis +40°C, durchgeführt. Die Umsetzungen können bei normalem, erhöhtem oder bei erniedrigtem Druck durchgeführt werden (z.B. von 0.5 bis 5 bar). Im Allgemeinen arbeitet man bei Normaldruck.

Der Verfahrensschritt (IV) + (V) → (I) wird im Allgemeinen in einem Temperaturbereich von +20°C bis +100°C, bevorzugt von +50°C bis +80°C, durchgeführt. Die Umsetzung kann bei normalem, erhöhtem oder bei erniedrigtem Druck durchgeführt werden (z.B. von 0.5 bis 5 bar). Im Allgemeinen arbeitet man bei Normaldruck.

Inerte Lösungsmittel für den Verfahrensschritt (VI) + (II) → (I) sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder andere Lösungsmittel wie Ethylacetat, Pyridin, Dimethylsulfoxid, Dimethylformamid, *N,N'*-Dimethylpropylenharnstoff (DMPU), *N*-Methylpyrrolidon (NMP), Acetonitril oder Aceton. Ebenso ist es möglich, Gemische der genannten Lösungsmittel zu verwenden. Bevorzugt sind Dichlormethan, Tetrahydrofuran, Dimethylformamid oder Gemische dieser Lösungsmittel.

Als Kondensationsmittel für die Amidbildung im Verfahrensschritt (VI) + (II) → (I) eignen sich beispielsweise Carbodiimide wie *N,N*'-Diethyl-, *N,N'*-Dipropyl-, *N,N'*-Diisopropyl-, *N,N*'-Dicyclohexylcarbodiimid (DCC), *N*-(3-Dimethylaminoisopropyl)-*N'*-ethylcarbodiimid-Hydrochlorid (EDC), oder Phosgen-Derivate wie *N,N'*-Carbonyldiimidazol, oder 1,2-Oxazoliumverbindungen wie 2-Ethyl-5-phenyl-1,2-oxazolium-3-sulfat oder 2-*tert*.-Butyl-5-methyl-isoxazolium-perchlorat, oder Acylaminoverbindungen wie 2-Ethoxy-1-ethoxycarbonyl-1,2-dihydrochinolin, oder Isobutylchlorformiat, Propanphosphonsäureanhydrid (PPA), Cyanophosphonsäurediethylester, Bis-(2-oxo-3-oxazolidinyl)-phosphorylchlorid, Benzotriazol-1-yloxy-tris(dimethylamino)phosphonium-hexafluorophosphat, Benzotriazol-1-yloxy-tris(pyrrolidino)phosphonium-hexafluorophosphat (PyBOP), *O*-(Benzotriazol-1-yl)-*N,N,N',N'*-tetramethyluronium-tetrafluoroborat (TBTU), *O*-(Benzotriazol-1-yl)-*N,N,N',N'*-tetramethyluronium-hexafluorophosphat (HBTU), 2-(2-Oxo-1-(2*H*)-pyridyl)-1,1,3,3-tetramethyluronium-tetrafluoroborat (TPTU), *O*-(7-Azabenzotriazol-1-yl)-*N,N,N',N'*-tetramethyl-uronium-hexafluorophosphat (HATU) oder *O*-(1*H*-6-Chlorbenzotriazol-1-yl)-1,1,3,3-tetramethyl-uronium-tetrafluoroborat (TCTU), gegebenenfalls in Kombination mit weiteren Hilfsstoffen wie 1-Hydroxybenzotriazol (HOBt) oder *N*-Hydroxysuccinimid (HOSu), sowie als Basen Alkalicarbonate, z.B. Natrium- oder Kaliumcarbonat oder -hydrogencarbonat, oder organische Basen wie Trialkylamine, z.B. Triethylamin, *N*-Methylmorpholin, *N*-Methylpiperidin oder *N,N*-Diisopropylethylamin. Bevorzugt werden TBTU, HATU oder PPA, jeweils in Kombination mit *N,N*-Diisopropylethylamin, verwendet.

Der Verfahrensschritt (VI) + (II) → (I) wird im Allgemeinen in einem Temperaturbereich von -20°C bis +60°C, bevorzugt von 0°C bis +40°C, durchgeführt. Die Umsetzung kann bei normalem, erhöhtem oder bei erniedrigtem Druck durchgeführt werden (z.B. von 0.5 bis 5 bar). Im Allgemeinen arbeitet man bei Normaldruck.

Die Verbindungen der Formeln (II), (III) und (V) sind kommerziell erhältlich, literaturbekannt oder können in Analogie zu literaturbekannten Verfahren hergestellt werden.

Die Herstellung der erfindungsgemäßen Verbindungen kann durch das folgende Syntheseschema veranschaulicht werden:

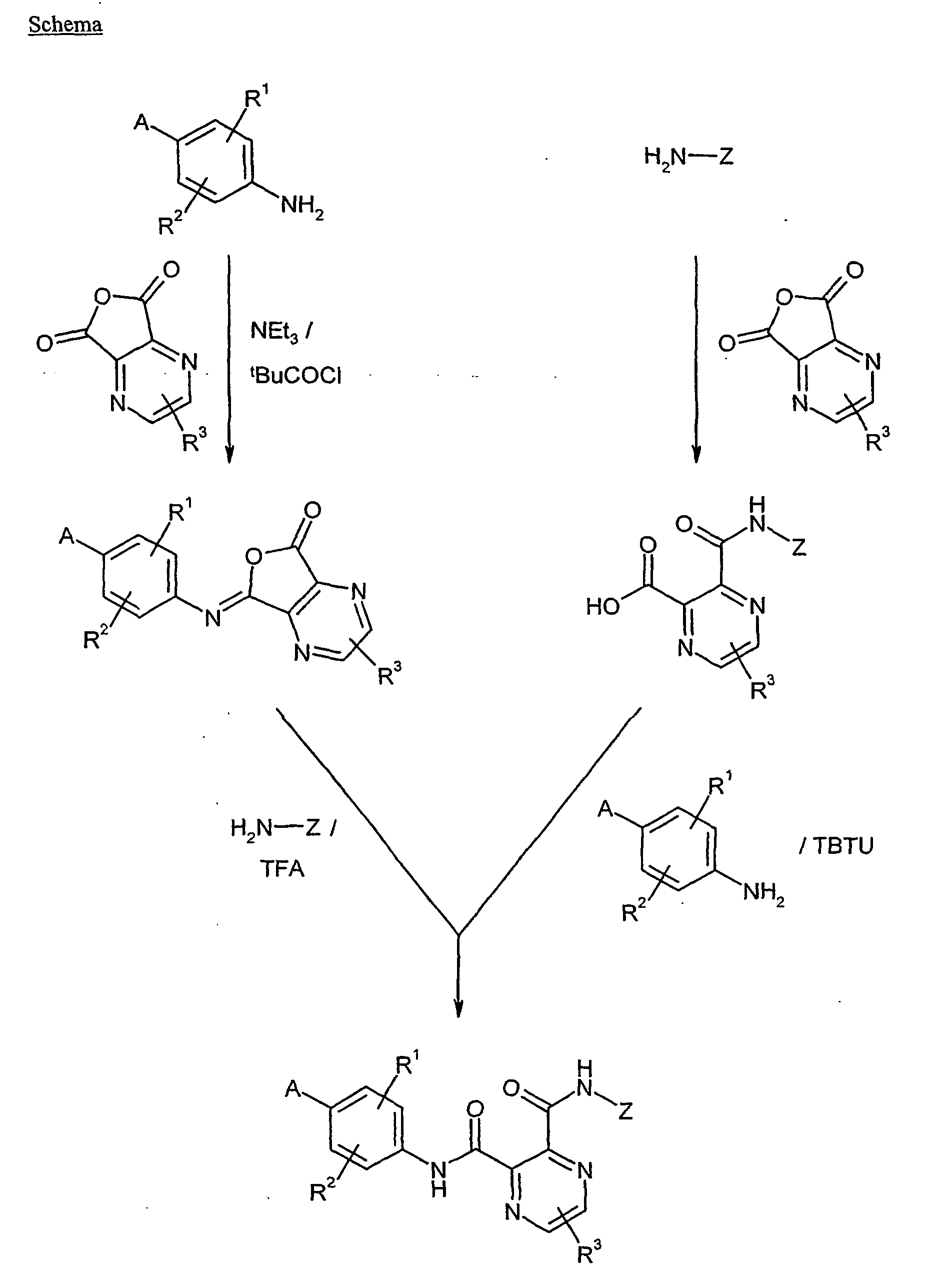

[Abkürzungen: ^{t}Bu = *tert*.-Butyl; Et = Ethyl; TBTU = *O*-(Benzotriazol-1-yl)-*N,N,N,N'*-tetramethyl-uronium-tetrafluoroborat; TFA = Trifluoressigsäure].

Die erfindungsgemäßen Verbindungen zeigen ein nicht vorhersehbares, wertvolles pharmakologisches Wirkspektrum, insbesondere eine hohe Wirkstärke sowie eine günstige Halbwertszeit.

Sie eignen sich daher zur Verwendung als Arzneimittel zur Behandlung und/oder Prophylaxe von Krankheiten bei Menschen und Tieren.

Bei den erfindungsgemäßen Verbindungen handelt es sich um selektive Inhibitoren des Blutgerinnungsfaktors Xa, die insbesondere als Antikoagulantien wirken.

Weiterer Gegenstand der vorliegenden Erfindung ist der Einsatz der erfindungsgemäßen Verbindungen zur Behandlung und/oder Prophylaxe von Erkrankungen, vorzugsweise von thromboembolischen Erkrankungen und/oder thromboembolischen Komplikationen.

Zu den "thromboembolischen Erkrankungen" im Sinne der vorliegenden Erfindung zählen insbesondere Erkrankungen wie Herzinfarkt mit ST-Segment-Erhöhung (STEMI) und ohne ST-Segment-Erhöhung (non-STEMI), stabile Angina Pectoris, instabile Angina Pectoris, Reokklusionen und Restenosen nach Koronarinterventionen wie Angioplastie oder aortokoronarem Bypass, periphere arterielle Verschlusskrankheiten, Lungenembolien, tiefe venöse Thrombosen und Nierenvenenthrombosen, transitorische ischämische Attacken sowie thrombotischer und thromboembolischer Hirnschlag.

Die Substanzen eignen sich daher auch zur Prävention und Behandlung von kardiogenen Thromboembolien, wie beispielsweise Hirn-Ischämien, Schlaganfall und systemischen Thromboembolien und Ischämien, bei Patienten mit akuten, intermittierenden oder persistierenden Herzarrhythmien, wie beispielsweise Vorhofflimmern, und solchen, die sich einer Kardioversion unterziehen, ferner bei Patienten mit Herzklappen-Erkrankungen oder mit künstlichen Herzklappen. Darüber hinaus sind die erfindungsgemäßen Verbindungen zur Behandlung der disseminierten intravasalen Gerinnung (DIC) geeignet.

Thromboembolische Komplikationen treten ferner auf bei mikroangiopathischen hämolytischen Anämien, extrakorporalen Blutkreisläufen, wie Hämodialyse, sowie Herzklappenprothesen.

Außerdem kommen die erfindungsgemäßen Verbindungen auch für die Prophylaxe und/oder Behandlung von atherosklerotischen Gefäßerkrankungen und entzündlichen Erkrankungen wie rheumatische Erkrankungen des Bewegungsapparats in Betracht, darüber hinaus ebenso für die Prophylaxe und/oder Behandlung der Alzheimer'schen Erkrankung. Außerdem können die erfindungsgemäßen Verbindungen zur Inhibition des Tumorwachstums und der Metastasenbildung, bei Mikroangiopathien, altersbedingter Makula-Degeneration, diabetischer Retinopathie, diabetischer Nephropathie und anderen mikrovaskulären Erkrankungen sowie zur Prävention und Behandlung thromboembolischer Komplikationen, wie beispielsweise venöser Thromboembolien, bei Tumorpatienten, insbesondere solchen, die sich größeren chirurgischen Eingriffen oder einer Chemo- oder Radiotherapie unterziehen, eingesetzt werden.

Die erfindungsgemäßen Verbindungen können darüber hinaus auch zur Verhinderung von Koagulation *ex vivo* eingesetzt werden, z.B. zur Konservierung von Blut- und Plasmaprodukten, zur Reinigung/Vorbehandlung von Kathetern und anderen medizinischen Hilfsmitteln und Geräten, zur Beschichtung künstlicher Oberflächen von *in vivo* oder *ex vivo* eingesetzten medizinischen Hilfsmitteln und Geräten oder bei biologischen Proben, die Faktor Xa enthalten.

Weiterer Gegenstand der vorliegenden Offenbarung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Offenbarung ist ein Verfahren zur Behandlung und/oder Prophylaxe von Erkrankungen, insbesondere der zuvor genannten Erkrankungen, unter Verwendung einer antikoagulatorisch wirksamen Menge der erfindungsgemäßen Verbindung.

Weiterer Gegenstand der vorliegenden Erfindung ist ein Verfahren zur Verhinderung der Blutkoagulation *in vitro,* insbesondere bei Blutkonserven oder biologischen Proben, die Faktor Xa enthalten, das dadurch gekennzeichnet ist, dass eine antikoagulatorisch wirksame Menge der erfindungsgemäßen Verbindung zugegeben wird.

Weiterer Gegenstand der vorliegenden Erfindung sind Arzneimittel, enthaltend eine erfindungsgemäße Verbindung und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- Lipidsenker, insbesondere HMG-CoA-(3-Hydroxy-3-methylglutaryl-Coenzym A)-Reduktase-Inhibitoren;
- Koronartherapeutika/Vasodilatatoren, insbesondere ACE-(Angiotensin-Converting-Enzyme)-Inhibitoren; AII-(Angiotensin II)-Rezeptor-Antagonisten; β-Adrenozeptor-Antagonisten; alpha-1-Adrenozeptor-Antagonisten; Diuretika; Calciumkanal-Blocker; Substanzen, die eine Erhöhung von cyclischem Guanosinmonophosphat (cGMP) bewirken, wie beispielsweise Stimulatoren der löslichen Guanylatcyclase;
- Plasminogen-Aktivatoren (Thrombolytika/Fibrinolytika) und die Thrombolyse/Fibrinolyse steigernde Verbindungen wie Inhibitoren des Plasminogen-Aktivator-Inhibitors (PAI-Inhibitoren) oder Inhibitoren des Thrombin-aktivierten Fibrinolyse-Inhibitors (TAFI-Inhibitoren);
- antikoagulatorisch wirksame Substanzen (Antikoagulantien);
- plättchenaggregationshemmende Substanzen (Plättchenaggregationshemmer, Thrombozytenaggregationshemmer);
- Fibrinogen-Rezeptor-Antagonisten (Glycoprotein-IIb/IIIa-Antagonisten);
- sowie Antiarrhythmika.

Weiterer Gegenstand der vorliegenden Erfindung sind Arzneimittel, die mindestens eine erfindungsgemäße Verbindung, üblicherweise zusammen mit einem oder mehreren inerten, nicht-toxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder -Augen-präparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- DCI: direkte chemische Ionisation (bei MS)
- DMF: *N,N-*Dimethylformamid
- DMSO: Dimethylsulfoxid
- d. Th.: der Theorie (bei Ausbeute)
- ee: Enantiomerenüberschuss
- eq.: Äquivalent(e)
- ESI: Elektrospray-Ionisation (bei MS)
- h: Stunde(n)
- HPLC: Hochdruck-, Hochleistungsflüssigchromatographie
- LC-MS: Flüssigchromatographie-gekoppelte Massenspektroskopie
- min: Minute(n)
- MS: Massenspektroskopie
- NMR: Kernresonanzspektroskopie
- RP: reverse phase (bei HPLC)
- RT: Raumtemperatur
- Rₜ: Retentionszeit (bei HPLC)
- THF: Tetrahydrofuran

### LC-MS- und HPLC-Methoden:

### Methode 1:

Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 11 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min, 2.5 min/3.0 min/4.5 min 2 ml/min; Ofen: 50°C; UV-Detektion: 210 nm.

### Methode 2:

Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min, 2.5 min/3.0 min/4.5 min 2 ml/min; Ofen: 50°C; UV-Detektion: 210 nm.

### Methode 3:

Instrument: Micromass Quattro LCZ mit HPLC Agilent Serie 1100; Säule: Phenomenex Synergi 2µ Hydro-RP Mercury 20 mm x 4 mm; Eluent A: 1 1 Wasser + 0.5 ml 50%-ige Ameisensäure, Eluent B: 1 l Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min, 2.5 min/3.0 min/4.5 min 2 ml/min; Ofen: 50°C; UV-Detektion: 208-400 nm.

### Methode 4:

Instrument: Micromass Platform LCZ mit HPLC Agilent Serie 1100; Säule: Phenomenex Synergi 2µ Hydro-RP Mercury 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min, 2.5 min/3.0 min/4.5 min 2 ml/min; Ofen: 50°C; UV-Detektion: 210 nm.

### Methode 5:

Instrument: Micromass Platform LCZ mit HPLC Agilent Serie 1100; Säule: Thermo HyPURITY Aquastar 3µ 50 mm x 2.1 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 0.2 min 100% A → 2.9 min 30% A → 3.1 min 10% A → 5.5 min 10% A; Ofen: 50°C; Fluss: 0.8 ml/min; UV-Detektion: 210 nm.

### Methode 6:

Gerätetyp MS: Micromass ZQ; Gerätetyp HPLC: Waters Alliance 2795; Säule: Merck Chromolith SpeedROD RP-18e 50 mm x 4.6 mm; Eluent A: 11 Wasser + 0.5 ml 50%-ige Ameisensäure, Eluent B: 1 l Acetonitril + 0.5 ml 50%-ige Ameisensäure; Gradient: 0.0 min 10% B → 3.0 min 95% B → 4.0 min 95% B; Ofen: 35°C; Fluss: 0.0 min 1.0 ml/min → 3.0 min 3.0 ml/min → 4.0 min 3.0 ml/min; UV-Detektion: 210 nm.

### Methode 7:

Instrument: HP 1100 mit DAD-Detektion; Säule: Kromasil 100 RP-18, 60 mm x 2.1 mm, 3.5 µm; Eluent A: 5 ml HClO₄ (70%-ig) / 1 Wasser, Eluent B: Acetonitril; Gradient: 0 min 2% B → 0.5 min 2% B → 4.5 min 90% B → 9 min 0% B → 9.2 min 2% B → 10 min 2% B; Fluss: 0.75 ml/min; Säulentemperatur: 30°C; UV-Detektion: 210 nm.

### Methode 8:

Instrument: HP 1100 mit DAD-Detektion; Säule: Kromasil 100 RP-18, 60 mm x 2.1 mm, 3.5 µm; Eluent A: 5 ml HClO₄ (70%-ig) / l Wasser, Eluent B: Acetonitril; Gradient: 0 min 2% B → 0.5 min 2% B → 4.5 min 90% B → 15 min 90% B → 15.2 min 2% B → 16 min 2% B; Fluss: 0.75 ml/min; Säulentemperatur: 30°C; UV-Detektion: 210 nm.

### Methode 9:

Instrument: HP 1100 mit DAD-Detektion; Säule: Kromasil 100 RP-18, 60 mm x 2.1 mm, 3.5 µm; Eluent A: 5 ml HClO₄ (70%-ig) / 1 Wasser, Eluent B: Acetonitril; Gradient: 0 min 2% B → 0.5 min 2% B → 4.5 min 90% B → 6.5 min 90% B → 6.7 min 2% B → 7.5 min 2% B; Fluss: 0.75 ml/min; Säulentemperatur: 30°C; UV-Detektion: 210 nm.

### Ausgangsverbindungen und Intermediate:

### Beispiel 1A

### 1-(4-Aminophenyl)pyrrolidin-2-on

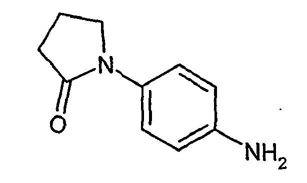

Die Darstellung erfolgt durch Reduktion von 1-(4-Nitrophenyl)-2-pyrrolidinon, siehe Reppe et al., Justus Liebigs Ann. Chem. 1955, 596, 209.

### Beispiel 2A

### 3-(4-Aminophenyl)-1,3-oxazolidin-2-on

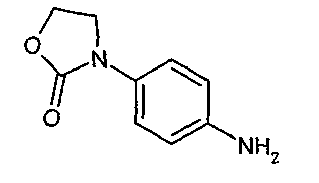

Die Darstellung erfolgt auf literaturbekanntem Weg, siehe M. Artico et al., Farmaco Ed. Sci. 1969, 24, 179-190.

### Beispiel 3A

### 1-(4-Aminophenyl)imidazolidin-2-on

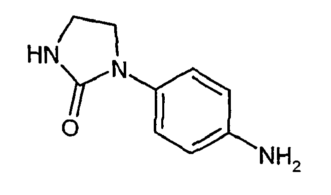

2.0 g (9.6 mmol) 1-(4-Nitrophenyl)imidazolidin-2-on [dargestellt durch Mitsunobu-Reaktion von 1-(2-Hydroxyethyl)-3-(4-nitrophenyl)harnstoff, siehe T.H. Kim, G.J. Lee, M.-H. Cha, Synth. Commun. 1999, 29, 2753-2758] werden in 20 ml DMF/THF (1:1) gelöst, mit 200 mg Palladium auf Aktivkohle (5%-ig) versetzt und in einer Wasserstoffatmosphäre bei RT und Normaldruck hydriert. Nach 12 h wird das Reaktionsgemisch mit Tonsil über Celite abgesaugt, mit THF gewaschen, das Filtrat eingeengt und der Rückstand im Hochvakuum getrocknet.
Ausbeute: 1.7 g (93% d. Th.)
LC-MS (Methode 7): Rₜ = 0.31 min;
MS (ESIpos): m/z = 178 [M+H]⁺.

### Beispiel 4A

### 1-(4-Amino-3-fluorphenyl)-3-hydroxypiperidin-2-on

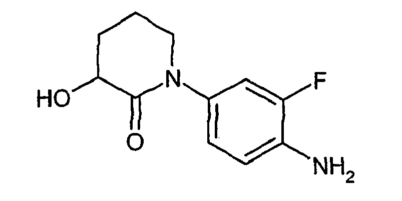

Die Darstellung erfolgt analog einer literaturbekannten Methode [A. Klapers et al., J. Am. Chem. Soc. 2002, 124, 7421-7428] aus 2-Fluor-4-iodanilin und 3-Hydroxypiperidin-2-on [Darstellung siehe I.S. Hutchinson et al., Tetrahedron 2002, 58, 3137-3143]:

Eine Suspension aus 6.45 g (27.2 mmol) 2-Fluor-4-iodanilin, 3.92 g (34.0 mmol, 1.25 eq.) 3-Hydroxypiperidin-2-on, 1.04 g (5.5 mmol, 0.2 eq.) Kupfer(I)iodid, 11.56 g (54.5 mmol, 2 eq.) Kaliumphosphat und 1.2 ml (10.9 mmol, 0.4 eq.) *N*,*N*-Dimethylethylendiamin in 157 ml Dioxan wird unter Argon über Nacht unter Rückfluss gerührt. Es werden weitere 1.04 g (5.5 mmol, 0.2 eq.) Kupfer(I)iodid und 0.9 ml (8.2 mmol, 0.3 eq.) *N,N-*Dimethylethylendiamin nachgegeben, und das Reaktionsgemisch wird weitere 8h lang unter Rückfluss gerührt. Die Suspension wird über eine Kieselgur-Schicht filtriert und der Rückstand mit einer Mischung aus Dichlormethan und Methanol (1:1) gewaschen. Die vereinigten Filtrate werden im Vakuum eingeengt. Das Rohprodukt wird mittels Flash-Chromatographie gereinigt (Kieselgel 60, Eluent: Dichlormethan/Methanol 100:1 → 40:1).
Ausbeute: 2.57 g (41 % d. Th.)
HPLC (Methode 9): Rₜ = 1.52 min;
MS (DCI, NH₃): m/z = 242 [M+NH₄]⁺;
¹H-NMR (300 MHz, DMSO-d₆): δ = 6.94 (d, 1H), 6.81-6.65 (m, 2H), 5.12 (br. s, 2H), 3.99 (dt, 1H), 3.63-3.39 (m, 2H), 2.12-2.00 (m, 1H), 2.00-1.62 (m, 4H).

### Beispiel 5A

### tert.-Butyl [1-(4-aminophenyl)-2-oxopiperidin-3-yl]carbamat

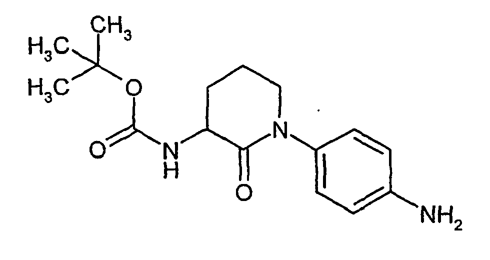

Die Darstellung erfolgt analog einer literaturbekannten Methode [A. Klapers et al., J. Am. Chem. Soc. 2002, 124, 7421-7428] aus 7.48 g (34.2 mmol) 4-Iodanilin und 9.00 g (42.0 mmol, 1.23 eq.) *tert.*-Butyl (2-oxopiperidin-3-yl)carbamat [Darstellung siehe K.-L. Yu et al., J. Med. Chem. 1988, 31, 1430-1436]; siehe auch Beispiel 4A.
Ausbeute: 6.4 g (60% d. Th.)
HPLC (Methode 9): Rₜ = 3.50 min;
MS (ESIpos): m/z= 306 [M+H]⁺;
¹H-NMR (300 MHz, DMSO-d₆): δ = 6.85 (d, 1H), 6.52 (d, 2H), 5.00 (s, 2H), 4.10-3.94 (m, 1H), 3.52-3.42 (m, 2H), 2.08-1.95 (m, 1H), 1.95-1.69 (m, 3H).

### Beispiel 6A

### 4-(4-Aminophenyl)morpholin-3-on

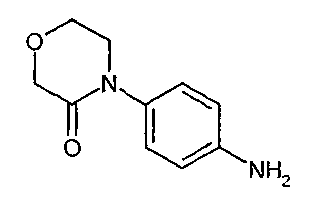

Die Darstellung erfolgt durch Substitution von 4-Fluornitrobenzol mit Morpholin-3-on [J.-M. Lehn, F. Montavon, Helv. Chim. Acta 1976, 59, 1566-1583] und anschließende Reduktion des 4-(4-Nitrophenyl)morpholin-3-ons (siehe WO O1/47919, Ausgangsverbindungen I bzw. n, S. 55-57).

### Beispiel 7A

### 4-(4-Amino-3-fluorphenyl)morpholin-3-on

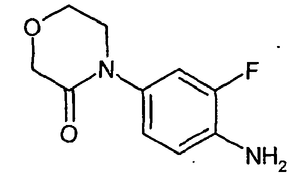

Die Darstellung erfolgt analog einer literaturbekannten Methode [A. Klapers et al., J. Am. Chem. Soc. 2002, 124, 7421-7428] aus 5.0 g (21.1 mmol) 2-Fluor-4-iodanilin und 2.6 g (26 mmol, 1.23 eq.) Morpholin-3-on; siehe auch Beispiel 4A.
Ausbeute: 4.2 g (94% d. Th.)
HPLC (Methode 2): Rₜ = 0.85 min;
MS (ESIpos): m/z = 211 [M+H]⁺;
¹H-NMR (300 MHz, DMSO-d₆): δ = 7.06 (dd, 1H), 6.87 (dd, 1H), 6.73 (dd, 1H), 5.18 (br. s, 2H), 4.14 (s, 2H), 3.92 (t, 2H), 3.62 (t, 2H).

### Beispiel 8A

### 4-(4-Aminophenyl)-2-(2-{[tert.-butyl(dimethyl)silyl]oxy}ethyl)morpholin-3-on

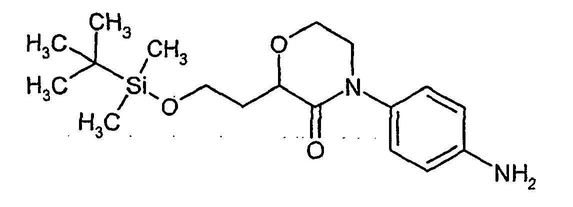

### Stufe a): 2-Allyl-4-(4-nitrophenyl)morpholin-3-on

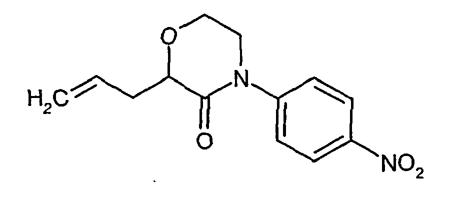

Zu einer Lösung von 13.7 g (81.9 mmol, 1.4 eq.) Lithium-1,1,1,3,3,3-hexamethyldisilazan-2-id in 400 ml THF werden bei -70°C unter Argon 13.0 g (58.5 mmol) 4-(4-Nitrophenyl)morpholin-3-on [J.-M. Lehn, F. Montavon, Helv. Chim. Acta 1976, 59, 1566-1583] gegeben. Das Reaktionsgemisch wird 10 min gerührt und dann mit 5.4 ml (58.5 mmol, 1.0 eq.) 3-Iod-1-propen, das zuvor über wenig Aluminiumoxid mit THF filtriert worden ist, versetzt. Das Reaktionsgemisch wird langsam auf RT kommen gelassen, auf ca. 100 ml Volumen eingeengt und mit einer Mischung aus Dichlormethan und Wasser versetzt. Nach Phasentrennung wird die wässrige Phase mit Dichlormethan extrahiert, die organischen Phasen über Magnesiumsulfat getrocknet, filtriert und im Vakuum eingeengt. Das Rohprodukt wird mittels Flash-Chromatographie gereinigt (Kieselgel 60, Eluent: Cyclohexan → Cyclohexan/Ethylacetat 1:1).
Ausbeute: 4.1 g (27% d. Th.)
LC-MS (Methode 1): Rₜ = 1.78 min;
MS (ESIpos): m/z = 263 [M+H]⁺.

### Stufe b): 2-(2-Hydroxyethyl)-4-(4-nitrophenyl)morpholin-3-on

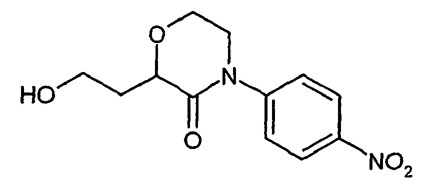

Eine Lösung von 1.33 g (5.07 mmol) 2-Allyl-4-(4-nitrophenyl)morpholin-3-on in 60 ml Tetrahydrofuran/Wasser (1:1) wird bei RT mit 0.62 ml (0.10 mmol, 0.02 eq.) einer 4%-igen wässrigen Osmiumtetroxid-Lösung und 3.25 g (15.2 mmol, 3 eq.) Natriumperiodat versetzt. Das Reaktionsgemisch wird 1.3 h bei RT gerührt und mit einer Mischung aus Wasser und Dichlormethan verdünnt. Nach Phasentrennung wird die wässrige Phase mit Dichlormethan extrahiert, und die vereinigten organischen Phasen werden über Magnesiumsulfat getrocknet, filtriert und im Vakuum eingeengt. Der Rückstand wird in 40 ml Tetrahydrofuran/Wasser (1:1) aufgenommen, die Reaktionslösung bei RT mit 96 mg (2.5 mmol, 0.5 eq.) Natriumborhydrid versetzt und 5 min bei RT gerührt. Nach Zugabe einer Mischung aus Wasser und Dichlormethan und Phasentrennung wird die wässrige Phase mit Dichlormethan extrahiert, und die vereinigten organischen Phasen werden über Natriumsulfat getrocknet, filtriert und im Vakuum eingeengt. Das Rohprodukt wird mittels Flash-Chromatographie gereinigt (Kieselgel 60, Eluent: Dichlormethan → Dichlormethan/Methanol 10:1).
Ausbeute: 1.1 g (80% d. Th.)
LC-MS (Methode 2): Rₜ = 1.49 min;
MS (ESIpos): m/z = 267 [M+H]⁺.

### Stufe c): 2-(2-{[tert.-Butyl(dimethyl)silyl]oxy}ethyl)-4-(4-nitrophenyl)morpholin-3-on

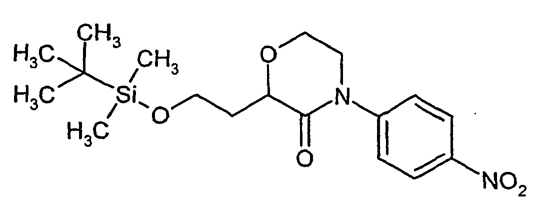

Eine Lösung aus 1.10 g (4.13 mmol) 2-(2-Hydroxyethyl)-4-(4-nitrophenyl)morpholin-3-on in 7 ml DMF wird bei RT mit 563 mg (8.26 mmol, 2 eq.) Imidazol und 934 mg (6.20 mmol, 1.5 eq.) *tert*.-Butyldimethylsilylchlorid versetzt und über Nacht bei 90°C gerührt. Das Reaktionsgemisch wird in gesättigte wässrige Natriumhydrogencarbonat-Lösung gegeben. Nach Zugabe von Diethylether und Phasentrennung wird die wässrige Phase mit Diethylether extrahiert, und die vereinigten organischen Phasen werden mit gesättigter wässriger Natriumchlorid-Lösung gewaschen, über Magnesiumsulfat getrocknet, filtriert und im Vakuum eingeengt. Das Rohprodukt wird ohne weitere Reinigung in der nächsten Stufe eingesetzt.
Ausbeute: 1.67 g (97% Reinheit)
LC-MS (Methode 1): Rₜ = 2.87 min;
MS (ESIpos): m/z = 381 [M+H]⁺.

### Stufe d): 4-(4-Aminophenyl)-2-(2-{[tert.-butyl(dimethyl)silyl]oxy}ethyl)morpholin-3-on

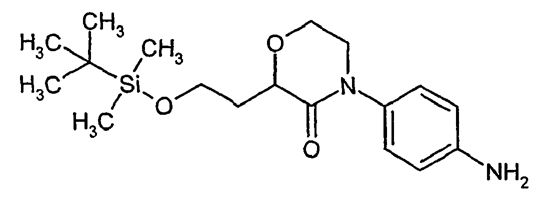

Eine Lösung aus 1.2 g (3.15 mmol) 2-(2-{[*tert*.-Butyl(dimethyl)silyl)oxy}ethyl)-4-(4-nitrophenyl)-morpholin-3-on in 15 ml Ethanol und 10 ml Wasser wird bei RT mit 5.0 ml einer 5%-igen wässrigen Eisentrichlorid-Lösung und 652 mg (11.7 mmol, 3.7 eq.) Eisenpulver versetzt und 2.5 h unter Rückfluss gerührt. Die Reaktionslösung wird heiß über Celite filtriert und im Vakuum eingeengt. Nach Zugabe von Wasser und Dichlormethan wird die Reaktionslösung mit einem Tropfen Ammoniak-Lösung alkalisch gestellt und nochmals über Celite filtriert. Nach Phasentrennung wird die wässrige Phase mit Dichlormethan extrahiert, und die vereinigten organischen Phasen werden über Magnesiumsulfat getrocknet, filtriert und im Vakuum eingeengt.
Ausbeute: 899 mg (95% Reinheit, 77% d. Th.)
LC-MS (Methode 1): Rₜ = 2.23 min;
MS (ESIpos): m/z = 351 [M+H]⁺;
¹H-NMR (300 MHz, CDCl₃): δ = 7.00 (d, 2H), 6.61 (d, 2H), 4.30 (dd, 1H), 4.08-3.98 (m, 1H), 3.87 (dd, 1H), 3.82-3.70 (m, 3H), 3.50-3.39 (m, 1H), 2.36-2.21 (m, 1H), 1.95-1.81 (m, 1H), 0.84 (s, 9H), 0.01 (s, 6H).

### Beispiel 9A

### 1-(4-Aminophenyl)-3-methyltetrahydropyrimidin-2(1H)-on

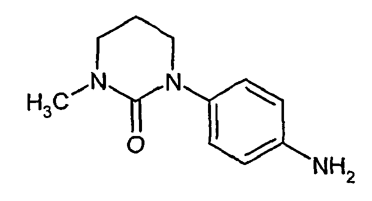

### Stufe a): 1-Methyl-3-(4-nitrophenyl)tetrahydropyrimidin-2(1H)-on

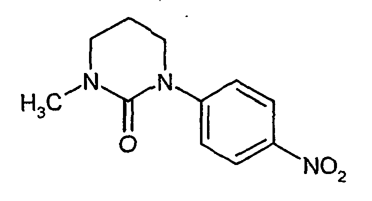

Eine Lösung aus 10.0 g (87.6 mmol) 1-Methyltetrahydropyrimidin-2(1*H*)-on [Darstellung siehe DE 1 121 617; Chem. Abstr. 1962, 56, 11601g] in 300 ml DMF wird unter Argon bei RT mit 14.8 g (131.4 mmol, 1.5 eq.) Kalium-*tert*.-butylat versetzt und 45 min bei RT gerührt. Anschließend wird das Reaktionsgemisch portionsweise mit 14.8 g (105.1 mmol, 1.2 eq.) 1-Fluor-4-nitrobenzol versetzt, über Nacht bei RT gerührt und dann im Vakuum eingeengt. Der Rückstand wird in einer Mischung aus gesättigter wässriger Natriumhydrogencarbonat-Lösung und Ethylacetat aufgenommen. Nach Phasentrennung wird die wässrige Phase mit Ethylacetat extrahiert. Die vereinigten organischen Phasen werden mit gesättigter wässriger Natriumchlorid-Lösung gewaschen, über Natriumsulfat getrocknet, filtriert und im Vakuum eingeengt. Der Rückstand wird in Toluol verrührt, abfiltriert und im Vakuum getrocknet.
Ausbeute: 9.4 g (46% d. Th.)
LC-MS (Methode 2): Rₜ = 1.68 min;
MS (ESIpos): m/z = 236 [M+H]⁺;
¹H-NMR (300 MHz, DMSO-d₆): δ = 8.14 (d, 2H), 7.55 (d, 2H), 3.77 (t, 2H), 3.38 (t, 2H), 2.90 (s, 3H), 2.05 (t, 2H).

### Stufe b): 1-(4-Aminophenyl)-3-methyltetrahydropyrimidin-2(1H)-on

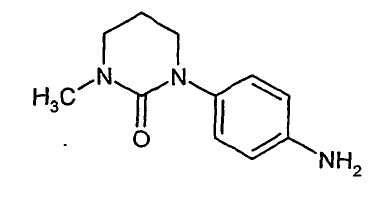

Eine Lösung aus 10.0 g (42.5 mmol) 1-Methyl-3-(4-nitrophenyl)tetrahydropyrimidin-2(1*H*)-on in 400 ml THF wird unter Argon mit 2.0 g Palladium auf Aktivkohle (5%-ig) versetzt und in einer Wasserstoffatmosphäre bei RT und Normaldruck über Nacht hydriert. Das Reaktionsgemisch wird mit Tonsil über Celite abgesaugt, mit Methanol gewaschen und das Filtrat im Vakuum eingeengt und getrocknet.
Ausbeute: 8.6 g (99% d. Th.)
LC-MS (Methode 5): Rₜ = 2.00 min;
MS (ESIpos): m/z = 206 [M+H]⁺;
¹H-NMR (300 MHz, DMSO-d₆): δ = 6.82 (d, 2H), 6.48 (d, 2H), 4.88 (br. s, 2H), 3.49 (t, 2H), 3.28 (t, 2H), 2.80 (s, 3H), 1.98 (quintett, 2H).

### Beispiele 10A

### 1-(4-Aminophenyl)-3-(2-hydroxyethyl)tetrahydropyrimidin-2(1H)-on

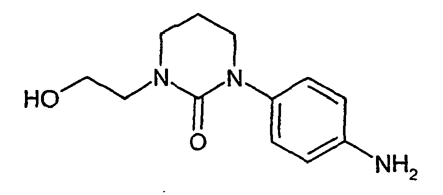

### Stufe a): 1-(2-Hydroxyethyl)-3-(4-nitrophenyl)tetrahydropyrimidin-2(1H)-on

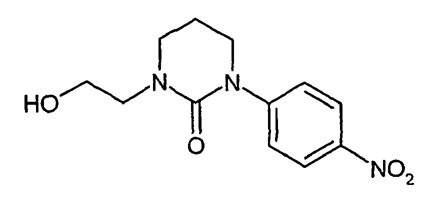

Die Darstellung erfolgt analog einer literaturbekannten Methode [A. Klapers et al., J. Am. Chem. Soc. 2002, 124, 7421-7428] aus 5.00 g (20.1 mmol) 1-Iod-4-nitrobenzol und 3.56 g (24.7 mmol, 1.23 eq.) 1-(2-Hydroxyethyl)tetrahydropyrimidin-2(1*H*)-on [Darstellung siehe DE 1 121 617; Chem. Abstr. 1962, 56, 11601g]; siehe auch Beispiel 4A.
Ausbeute: 2.93 g (51 % d. Th.)
LC-MS (Methode 2): Rₜ = 1.49 min;
MS (ESIpos): m/z = 266 [M+H]⁺.

### Stufe b): 1-(4-Aminophenyl)-3-(2-hydroxyethyl)tetrahydropyrimidin-2(1H)-on

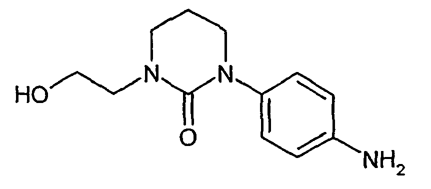

Eine Lösung aus 200 mg (0.75 mmol) 1-(2-Hydroxyethyl)-3-(4-nitrophenyl)tetrahydropyrimidin-2(1*H*)-on in 10 ml Methanol wird unter Argon mit 35 mg Palladium auf Aktivkohle (10%-ig) versetzt und in einer Wasserstoffatmosphäre bei RT und Normaldruck für 2 h hydriert. Der Katalysator wird über eine Kieselgel-Schicht abgetrennt und das Filtrat im Vakuum eingeengt. Der Rückstand wird in Diethylether verrührt, abfiltriert und im Vakuum getrocknet.
Ausbeute: 156 mg (88% d. Th.)
HPLC (Methode 9): Rₜ = 2.47 min;
MS (ESIpos): m/z = 236 [M+H]⁺;
¹H-NMR (300 MHz, DMSO-d₆): δ = 6.84 (d, 2H), 6.48 (d, 2H), 4.92 (s, 1H), 3.54-3.43 (m, 4H), 3.39 (t, 2H), 3.29 (t, 2H), 1.95 (q, 2H).

### Beispiel 11A

### 1-(4-Amino-3-fluorphenyl)-3-(hydroxymethyl)pyridin-2(1H)-on

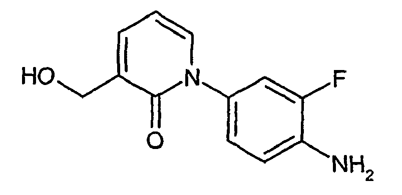

Die Darstellung erfolgt analog der Synthese von Beispiel 4A aus 5.39 g (22.8 mmol) 4-Iod-2-fluoranilin und 4.00 g (28.5 mmol, 1.25 eq.) 3-(Hydroxymethyl)pyridin-2(1*H*)-on [Darstellung siehe S. McN. Sieburth et al., Chem. Commun. 1996, 19, 2249-2250].
Ausbeute: 3.46 g (65% d. Th.)
HPLC (Methode 9): Rₜ = 2.44 min;
MS (ESIpos): m/z = 235 [M+H]⁺;
¹H-NMR (300 MHz, DMSO-d₆): δ = 7.48 (m, 2H), 7.10 (dd, 1H), 6.89 (dd, 1H), 6.80 (t, 1H), 6.30 (t, 1H), 5.39 (s, 2H), 5.11 (t, 1H), 4.31 (d, 2H).

### Beispiel 12A

### 3-{[(5-Chlorpyridin-2-yl)amino]carbonyl}pyrazin-2-carbonsäure

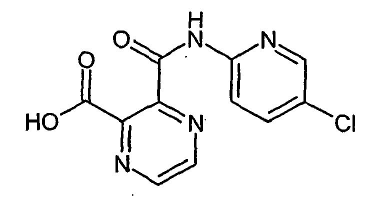

68.0 g (0.53 mol) 2-Amino-5-chlorpyridin werden in 1100 ml THF gelöst und portionsweise mit 95.3 g (0.63 mol) 2,3-Pyrazindicarbonsäureanhydrid versetzt. Die Suspension wird eine Stunde bei Raumtemperatur gerührt. Anschließend wird der Niederschlag abfiltriert. Das Filtrat wird eingeengt und der Rückstand mit dem Niederschlag vereinigt. Es wird in Diethylether verrührt, erneut filtriert und im Vakuum getrocknet.
Ausbeute: 154 g (99% d. Th.)
HPLC (Methode 9): Rₜ = 3.50 min;
MS (ESIpos): m/z = 279 [M+H]⁺;
¹H-NMR (300 MHz, DMSO-d₆): δ = 13.89 (br. s, 1H), 11.07 (s, 1H), 8.90 (dd, 2H), 8.44 (s, 1H), 8.20 (d, 1H), 8.01 (dd, 1H).

### Beispiele 13A

### 3-{[(4-Ethinylphenyl)amino]carbonyl}pyrazin-2-carbonsäure

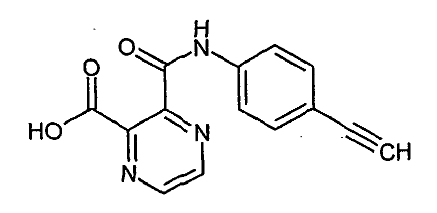

500 mg (4.27 mmol) 4-Ethinylanilin und 641 mg (4.27 mmol) 2,3-Pyrazindicarbonsäureanhydrid werden analog der für Beispiel 12A beschriebenen Methode umgesetzt.
Ausbeute: 1.08 g (96% Reinheit, 91% d. Th.)
HPLC (Methode 3): Rₜ = 1.49 min;
MS (ESIpos): m/z = 224 [M+H-CO₂]⁺.

### Beispiel 14A

### 3-{[(4-Chlorphenyl)amino]carbonyl pyrazin-2-carbonsäure

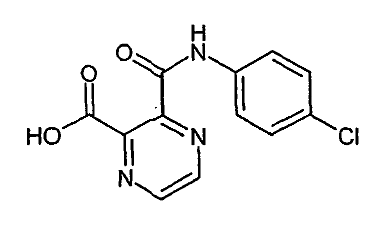

100 mg (0.78 mmol) 4-Chloranilin und 118 mg (0.78 mmol) 2,3-Pyrazindicarbonsäureanhydrid werden analog der für Beispiel 12A beschriebenen Methode umgesetzt.
Ausbeute: 115 mg (53% d. Th.)
HPLC (Methode 1): Rₜ = 1.28 min;
MS (ESIpos): m/z = 234 [M+H-CO₂]⁺;
¹H-NMR (300 MHz, DMSO-d₆): δ = 13.80 (br. s, 1H), 10.92 (s, 1H), 8.90 (dd, 2H), 7.82 (d, 2H), 7.44 (d, 2H).

### Beispiel 15A

### 7-{[4-(2-Oxo-1,3-oxazolidin-3-yl)phenyl]imino}furo[3,4-b]pyrazin-5(7H)-on

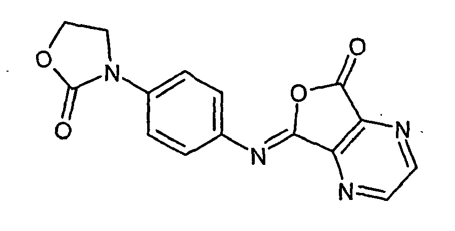

1.07 g (6 mmol) 3-(4-Aminophenyl)-1,3-oxazolidin-2-on (Beispiel 2A) werden unter Argon in 15 ml absolutem DMF gelöst. Dann werden 0.9 g (6 mmol) Pyrazindicarbonsäureanhydrid zugegeben und die Reaktionslösung 1 h gerührt. Nach Zugabe von 0.75 ml (0.55 g, 5.4 mmol) Triethylamin und 0.81 ml (0.8 g, 6.6 mmol) Pivaloylchlorid wird die Reaktionsmischung 0.5 h bei RT gerührt und danach mit Wasser verdünnt. Die Kristalle werden abgesaugt, mit Methanol und *tert*.-*Butylmethylether gewaschen und im Vakuum getrocknet.*
Ausbeute: 1.4 g (75% d. Th.)
LC-MS (Methode 3): Rₜ = 1.51 min;
MS (ESIpos): m/z = 311 [M+H]⁺;
¹H-NMR (300 MHz, DMSO-d₆): δ = 9.05 (s, 2H), 7.75 (d, 2H), 7.5 (d, 2H), 4.5 (tr, 2H), 4.15 (tr, 2H).

### Beispiel 16A

### 7-{[4-(2-Oxopyrrolidin-1-yl)phenyl]imino}furo[3,4-b]pyrazin-5(7H)-on

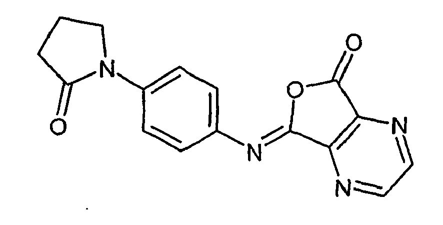

Die Darstellung erfolgt analog der Synthese von Beispiel 15A aus 1.06 g (6 mmol) 1 -(4-Amino- phenyl)pyrrolidin-2-on (Beispiel 1A) und 0.9 g (6 mmol) Pyrazindicarbonsäureanhydrid.
Ausbeute: 0.98 g (53% d. Th.)
LC-MS (Methode 3): Rₜ = 1.59 min;
MS (ESIpos): m/z = 309 [M+H]⁺;
¹H-NMR (300 MHz, DMSO-d₆): δ = 9.05 (s, 2H), 7.85 (d, 2H), 7.45 (d, 2H), 3.9 (tr, 2H), 2.55 (tr, 2H), 2.1 (quintett, 2H).

### Beispiel 17A

### 7-{[4-(2-Oxoimidazolidin-1-yl)phenyl] imino}furo[3,4-b]pyrazin-5(7H)-on

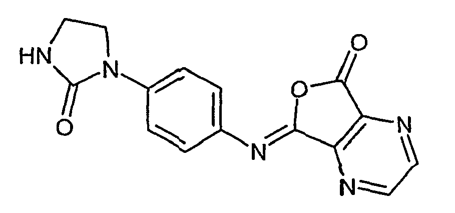

Die Darstellung erfolgt analog der Synthese von Beispiel 15A aus 1.32 g (7.45 mmol) 1-(4-Aminophenyl)imidazolidin-2-on (Beispiel 3A) und 1.12 g (7.45 mmol) Pyrazindicarbonsäureanhydrid.
Ausbeute: 1.08 g (47% d. Th.)
LC-MS (Methode 1): Rₜ = 1.18 min;
MS (ESIpos): m/z = 310 [M+H]⁺;
¹H-NMR (300 MHz, DMSO-d₆): δ = 9.05 (s, 2H), 7.75 (d, 2H), 7.4 (d, 2H), 7.05 (s, 1H), 3.9 (tr, 2H), 3.45 (tr, 2H).

### Beispiel 18A

### 7-{[4-(3-Oxomorpholin-4-yl)phenyl]imino}furo[3,4-b]pyrazin-5(7H)-on

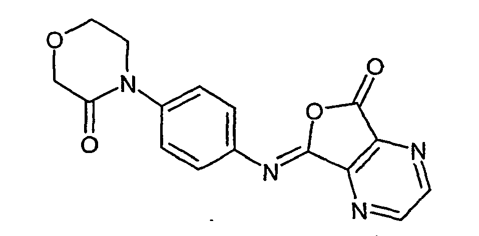

Die Darstellung erfolgt analog der Synthese von Beispiel 15A aus 1.15 g (6 mmol) 4-(4-Aminophenyl)morpholin-3-on (Beispiel 6A) und 0.9 g (6 mmol) Pyrazindicarbonsäureanhydrid.
Ausbeute: 1.3 g (69% d. Th.)
LC-MS (Methode 2): Rₜ = 1.30 min;
MS (ESIpos): m/z = 325 [M+H]⁺;
¹H-NMR (300 MHz, DMSO-d₆): δ = 9.1 (s, 2H), 7.6 (d, 2H), 7.5 (d, 2H), 4.25 (s, 2H), 4.05 (tr, 2H), 3.8 (dd, 2H).

### Beispiel 19A

### 7-{[4-(3-Methyl-2-oxotetrahydropyrimidin-1(2H)-yl)phenyl]imino}furo[3,4-b]pyrazin-5(7H)-on

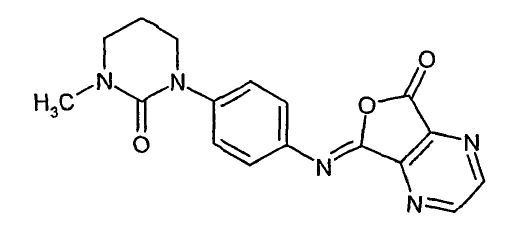

Die Darstellung erfolgt analog der Synthese von Beispiel 15A aus 1.23 g (6 mmol) 1-(4-Aminophenyl)-3-methyltetrahydropyrimidin-2(1*H*)-on (Beispiel 9A) und 0.9 g (6 mmol) Pyrazindicarbonsäureanhydrid.
Ausbeute: 1.3 g (64% d. Th.)
LC-MS (Methode 3): Rₜ = 1.56 min;
MS (ESIpos): m/z= 338 [M+H]⁺;
¹H-NMR (300 MHz, DMSO-d₆): δ = 9.05 (s, 2H), 7.45 (d, 2H), 7.35 (d, 2H), 3.75 (tr, 2H), 3.4 (tr, 2H), 2.9 (s, 3H), 2.1 (quintett, 2H).

### Beispiel 20A

### N-(2-{[tert.-Butyl(dimethyl)silyl]oxy}ethyl)benzol-1,4-diamin

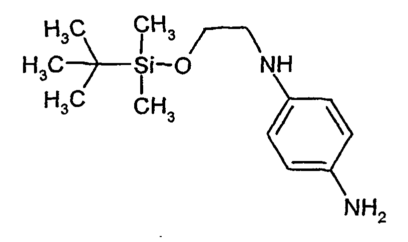

### Stufe a): 2-[(4-Nitrophenyl)amino]ethanol

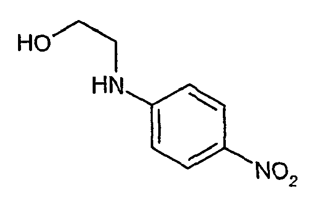

Eine Lösung aus 101 g (716 mmol) 4-Fluornitrophenol in 500 ml Ethanol wird mit 130 ml (2.15 mol, 3 eq.) 2-Aminoethanol und 274 ml (1,57 mol, 2.2 eq.) *N,N*-Diisopropylethylamin versetzt. Das Reaktionsgemisch wird bei 50°C über Nacht gerührt, anschließend mit weiteren 86 ml (1.43 mol, 2.0 eq.) 2-Aminoethanol und 249 ml (1.43 mol, 2.0 eq.) *N,N*-Diisopropylethylamin versetzt und weitere 12 h bei 50°C gerührt. Die Reaktionslösung wird im Vakuum eingeengt und der Rückstand mit 600 ml Wasser verrührt. Der entstandene Niederschlag wird abfiltriert, mehrmals mit Wasser gewaschen und getrocknet.
Ausbeute: 127 g (97% d. Th.)
LC-MS (Methode 5): Rₜ = 2.32 min;
MS (ESIpos): m/z =183 [M+H]⁺;
¹H-NMR (300 MHz, DMSO-d₆): δ = 7.99 (d, 2H), 7.30 (t, 1H), 6.68 (d, 2H), 4.82 (t, 1H), 3.63-3.52 (m, 2H), 3.30-3.19 (m, 2H).

### Stufe b): N-(2-{[tert.-Butyl(dimethyl)silyl]oxy}ethyl)-4-nitroanilin

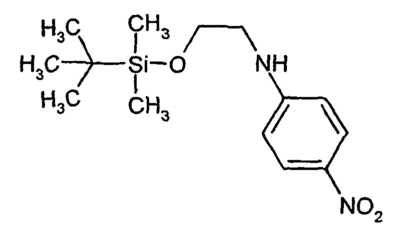

Eine Lösung aus 30.8 g (169 mmol) 2-[(4-Nitrophenyl)amino]ethanol in 300 ml DMF wird bei RT mit 30.6 g (203 mmol, 1.2 eq.) *tert.*-Butyldimethylchlorsilan und 17.3 g (254 mmol, 1.5 eq.) Imidazol versetzt und bei RT 2.5 h lang gerührt. Das Reaktionsgemisch wird im Vakuum eingeengt und der Rückstand in 200 ml Dichlormethan und 100 ml Wasser gelöst. Nach Phasentrennung wird die wässrige Phase dreimal mit jeweils 80 ml Dichlormethan extrahiert. Die vereinigten organischen Phasen werden mit 100 ml gesättigter wässriger Natriumchlorid-Lösung gewaschen, über Natriumsulfat getrocknet, filtriert und im Vakuum eingeengt.
Ausbeute: 49.7 g (quant.)
LC-MS (Methode 3): Rₜ = 3.09 min;
MS (ESIpos): m/z = 297 [M+H]⁺;
¹H-NMR (300 MHz, DMSO-d₆): δ = 7.98 (d, 2H), 7.29 (t, 1H), 6.68 (d, 2H), 3.77-3.66 (m, 2H), 3.35-3.24 (m, 2H), 0.81 (s, 9H), 0.0 (s, 6H).

### Stufe c): N-(2-{[tert.-Butyl(dimethyl)silyl]oxy}ethyl)benzol-1,4-diamin

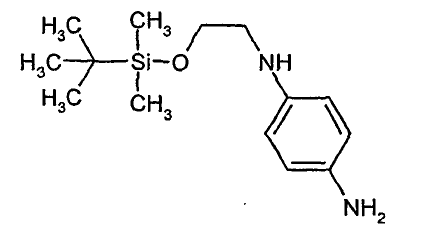

Eine Lösung aus 59.5 g (201 mmol) *N*-(2-{[*tert.*-Butyl(dimethyl)silyl]oxy}ethyl)-4-nitroanilin in 500 ml Ethanol wird unter Argon mit 4 g Palladium auf Aktivkohle (10%-ig) versetzt und in einer Wasserstoffatmosphäre bei RT und Normaldruck hydriert. Der Katalysator wird über eine Filterschicht abgetrennt, mit Ethanol gewaschen und das Filtrat im Vakuum eingeengt.
Ausbeute: 53 g (quant.)
LC-MS (Methode 2): Rₜ = 1.83 min;
MS (ESIpos): m/z = 267 [M+H]⁺;
¹H-NMR (300 MHz, DMSO-d₆): δ = 6.42-6.30 (m, 4H), 4.48 (t, 1H), 4.21 (br. s, 2H), 3.68-3.58 (m, 2H), 3.04-2.93 (m, 2H), 0.82 (s, 9H), 0.0 (s, 6H).

### Beispiel 21A

### 3-{[(4-Cyanophenyl)amino]carbonyl}pyrazin-2-carbonsäure

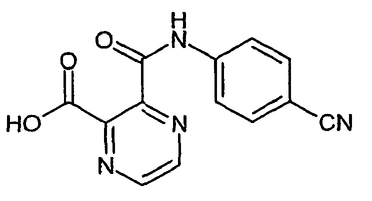

1.0 g (8.5 mmol) 4-Aminobenzonitril und 1.3 g (8.5 mmol) 2,3-Pyrazindicarbonsäureanhydrid werden analog der für Beispiel 12A beschriebenen Methode umgesetzt.
Ausbeute: 2.1g (92% d. Th.)
HPLC (Methode 3): Rₜ = 1.06 min;
MS (ESIpos): m/z = 225 [M+H-CO₂]⁺;
¹H-NMR (300 MHz, DMSO): δ = 13.92 (br. s, 1H), .11.22 (s, 1H), 8.92 (dd, 2H), 7.99 (d, 2H), 7.87 (d, 2H).

### Beispiel 22A

### 2-{2-[2-Amino-5-(3-oxomorpholin-4-yl)phenoxy]ethyl}-1H-isoindol-1,3(2H)-dion

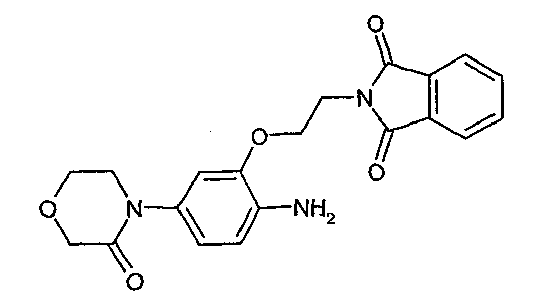

### Stufe a): 2-[2-(5-Fluor-2-nitrophenoxy)ethyl]-1H-isoindol-1,3(2H)-dion

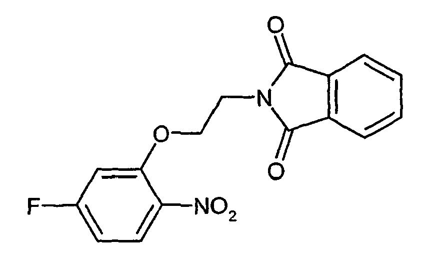

70.2 g (446.8 mmol) 5-Fluor-2-nitrophenol werden mit 130 ml DMF und 92.6 g (670.2 mmol, 1.5 eq.) Kaliumcarbonat versetzt. Dazu wird eine Lösung von 11.1 g (67.0 mmol, 0.15 eq.) Kaliumiodid und 113.5 g (446.8 mmol, 1.0 eq.) *N*-(2-Bromethyl)phthalimid in 200 ml DMF gegeben. Die Suspension wird 16 Stunden bei 80°C gerührt. Nach Abkühlen auf Raumtemperatur werden 1300 ml Wasser zugegeben. Es wird eine Stunde bei Raumtemperatur nachgerührt und anschließend der Feststoff abfiltriert. Der Rückstand wird je dreimal mit 200 ml Wasser und 200 ml Diethylether gewaschen. Der Feststoff wird bei 85°C im Hochvakuum getrocknet.
Ausbeute: 89.1 g (90% Reinheit, 54% d. Th.)
LC-MS (Methode 1): Rₜ = 2.09 min;
MS (ESIpos): m/z = 331 [M+H]⁺;
¹H-NMR (400 MHz, DMSO-d₆): δ = 7.97-7.93 (m, 1H), 7.91-7.82 (m, 4H), 7.38 (dd, 1H), 6.97 (dd, 1H), 4.45 (t, 2H), 4.00 (t, 2H).

### Stufe b): 2-{2-[2-Nitro-5-(3-oxomorpholin-4-yl)phenoxy]ethyl}-1H-isoindol-1,3(2H)-dion

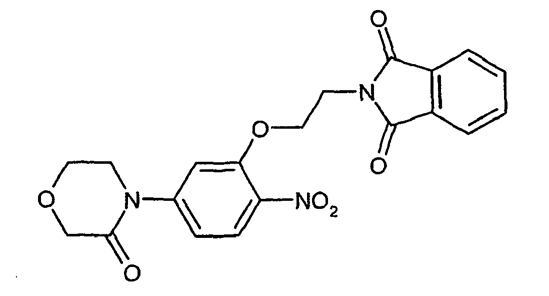

Eine Lösung von 1.81 g (17.91 mmol) Morpholin-3-on [E. Pfeil, U. Harder, Angew. Chem. 1967, 79, 188] in 100 ml DMF wird mit 3.02 g (26.87 mmol, 1.5 eq.) Kalium-tert.-butylat und 7.10 g (21.50 mmol, 1.2 eq.) 2-[2-(5-Fluor-2-nitrophenoxy)ethyl]-1*H*-isoindol-1,3(2*H*)-dion versetzt. Es wird 18 Stunden bei Raumtemperatur und dann 4 Stunden bei 80°C gerührt. Nach Abkühlen werden 400 ml Wasser sowie 200 ml Dichlormethan zugegeben. Die organische Phase wird abgetrennt und die wässrige Phase erneut zweimal mit je 100 ml Dichlormethan extrahiert. Die vereinigten organischen Phasen werden im Vakuum eingeengt. Der Rückstand wird säulenchromatographisch an Kieselgel (Laufmittel: Dichlormethan/Methanol 98:2) und anschließend mittels präparativer RP-HPLC gereinigt.
Ausbeute: 940 mg (13% d. Th.)
LC-MS (Methode 1): Rₜ = 1.73 min;
MS (ESIpos): m/z = 412 [M+H]⁺;
¹H-NMR (400 MHz, DMSO-d₆): δ = 7.91-7.84 (m, 5H), 7.47 (d, 1H), 7.20 (dd, 1H), 4.41 (t, 2H), 4.24 (s, 2H), 4.02-3.97 (m, 4H), 3.81 (t, 2H).

### Stufe c): 2-{2-[2-Amino-5-(3-oxomorpholin-4-yl)phenoxy]ethyl)-1H-isoindol-1,3(2H)-dion

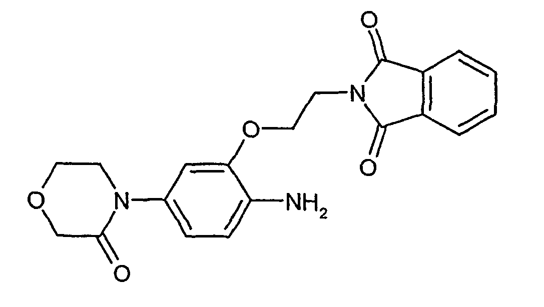

Eine mit Argon überschichtete Suspension von 935 mg (2.27 mmol) 2-{2-[2-Nitro-5-(3-oxomorpholin-4-yl)phenoxy]ethyl}-1*H*-isoindol-1,3(2*H*)-dion in 100 ml eines Ethanol/Methanol/Dichlormethan-Gemisches (1:1:1) wird mit 121 mg (0.11 mmol, 0.05 eq.) Palladium auf Kohle (10%-ig) versetzt. Es wird 16 Stunden bei Raumtemperatur und Normaldruck hydriert. Nach Filtration über Kieselgur wird die Lösung im Vakuum eingeengt und der Rückstand säulenchromatographisch an Kieselgel (Laufinittel: Essigsäureethylester/Methanol 98:2) aufgereinigt. Das erhaltene Produkt wird in Diethylether verrührt und im Hochvakuum getrocknet.
Ausbeute: 547 mg (78% Reinheit, 49% d. Th.)
LC-MS (Methode 1): Rₜ = 1.37 min;
MS (ESIpos): m/z= 3 82 [M+H]⁺.

### Ausführungsbeispiele:

### Allgemeine Methode 1: Öffnung der Phenyliminofuro[3,4-b]pyrazin-5(7H)-one

0.1 mmol Phenyliminofuro[3,4-b]pyrazin-5(7H)-on werden unter Argon in 0.15 ml absolutem DMF gelöst. Dann werden 7.7 µl (11.4 mg, 0.1 mmol) Trifluoressigsäure und 0.1 oder 0.2 mmol Anilin-Derivat hinzugegeben und die Reaktionsmischung über Nacht bei 70°C gerührt. Die Reaktionsmischung wird nach Abkühlen mit 0.1 ml DMF und wenig Wasser verdünnt und filtriert. Das Filtrat wird durch präparative HPLC gereinigt (Säule: Machery Nagel VP50/21 Nucleosil 100-5 C18 Nautilus, 5 µm, 21 x 50 mm; Injektionsvolumen: 500 µl; Eluent A = Wasser + 0.1% Ameisensäure, Eluent B = Acetonitril; Gradient: 0 min 10% B → 2 min 10% B → 6 min 90% B → 7 min 90% B → 7.1 min 10% B → 8 min 10% B; Flussrate: 25 ml/min; Wellenlänge: 220 nm). Die produkthaltigen Fraktionen werden im Vakuum eingeengt.

### Allgemeine Methode 2: Amid-Kupplung - Verfahren I

3-{[Arylamino]carbonyl}pyrazin-2-carbonsäure und *N,N*-Diisopropylethylamin (1.05 eq.) werden in Dichlormethan vorgelegt und 15 min bei RT gerührt. Anschließend wird eine Lösung des Anilin-Derivats (1.0 eq.) in Dichlormethan zugetropft. Man gibt *O*-(Benzotriazol-1-yl)-*N,N,N',N'-*tetramethyluroniumtetrafluoroborat (1.05 eq.) hinzu und rührt bei Raumtemperatur über Nacht. Anschließend wird die Reaktionslösung mit Wasser, mit gesättigter wässriger Natriumhydrogencarbonat-Lösung und erneut mit Wasser gewaschen. Das Lösungsmittel wird im Vakuum entfernt und der Rückstand mit Essigsäureethylester versetzt. Der ausgefallene Feststoff wird abfiltriert und mit Pentan gewaschen. Das Filtrat wird im Vakuum eingeengt und der Rückstand säulenchromatographisch gereinigt.

### Allgemeine Methode 3: Amid-Kupplung - Verfahren II

Eine Lösung des Anilin-Derivats (1.0 eq.) in Dichlormethan wird mit *N,N*-Diisopropylethylamin (10.0 eq.) versetzt. Anschließend werden 3-{[Arylamino]carbonyl}pyrazin-2-carbonsäure (1.1 eq.) und *n*-Propanphosphonsäureanhydrid (*n*-PPA) (2.0 eq.) hinzugegeben. Die Reaktionssuspension wird über Nacht bei RT gerührt. Das Lösungsmittel wird im Vakuum entfernt. Der Rückstand wird in DMSO aufgenommen und mittels RP-HPLC gereinigt (Eluent: Wasser/Acetonitril 90:10 → 2:98).

Nach der Allgemeinen Methode 1 werden die folgenden Verbindungen ausgehend von Beispiel 16A dargestellt:

### Beispiel 1

### N-(4-Chlorphenyl)-N'-[4-(2-oxopyrrolidin-1-yl)phenyl]pyrazin-2,3-dicarboxamid

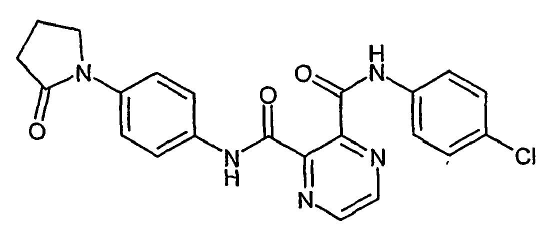

Ausbeute: 11.3 mg (26% d. Th.)
LC-MS (Methode 1): Rₜ = 1.84 min;
MS (ESIpos): m/z = 436 [M+H]⁺.

### Beispiel 2

### N-(5-Chlorpyridin-2-yl)-N'-[4-(2-oxopyrrolidin-1-yl)phenyl]pyrazin-2,3-dicarboxamid

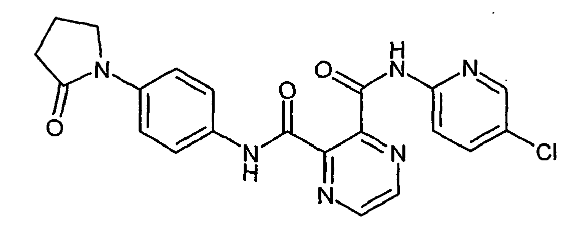

Ausbeute: 3 mg (7% d. Th.)
LC-MS (Methode 2): Rₜ = 1.96 min;
MS (ESIpos): m/z = 437 [M+H]⁺.

### Beispiel 3

### N-(6-Chlorpyridin-3-yl)-N'-[4-(2-oxopyrrolidin-1-yl)phenyl]pyrazin-2,3-dicarboxamid

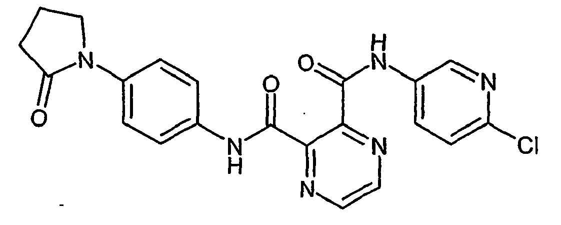

Ausbeute: 12 mg (27% d. Th.)
LC-MS (Methode 2): Rₜ =1.76 min;
MS (ESIpos): m/z = 437 [M+H]⁺.

### Beispiel 4

### N-(4-Fluorphenyl)-N'-[4-(2-oxopyrrolidin-1-yl)phenyl]pyrazin-2,3-dicarboxamid

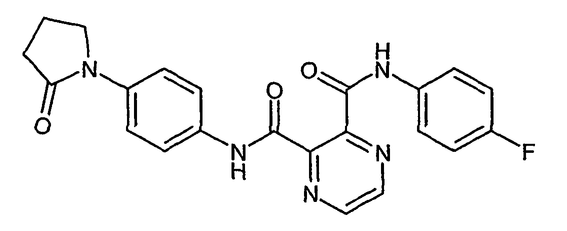

Ausbeute: 26 mg (62% d. Th.)
LC-MS (Methode 4): Rₜ = 2.07 min;
MS (ESIpos): m/z = 420 [M+H]⁺.

### Beispiel 5

### N-(4-Methylphenyl)-N'-[4-(2-oxopyrrolidin-1-yl)phenyl]pyrazin-2,3-dicarboxamid

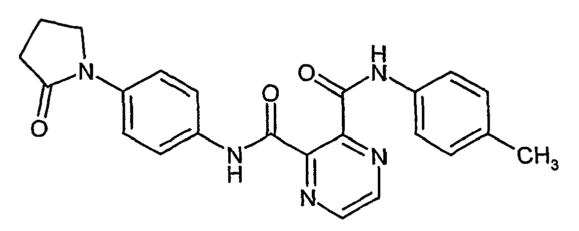

Ausbeute: 14 mg (34% d. Th.)
LC-MS (Methode 2): Rₜ = 1.98 min;
MS (ESIpos): m/z = 416 [M+H]⁺.

### Beispiel 6

### N-(4-Chlorphenyl)-N'-[4-(2-oxo-1,3-oxazolidin-3-yl)phenyl]pyrazin-2,3-dicarboxamid

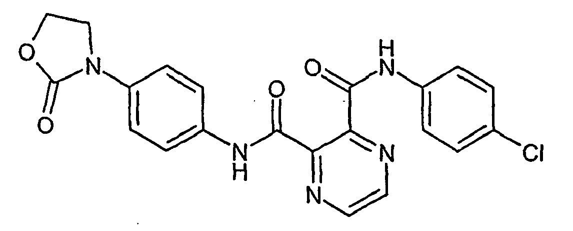

Nach der Allgemeinen Methode 1 wird die Titelverbindung ausgehend von Beispiel 15A dargestellt.
Ausbeute: 5 mg (11% d. Th.)
LC-MS (Methode 1): Rₜ = 1.82 min;
MS (ESIpos): m/z = 438 [M+H]⁺.

### Beispiel 7

### N-(5-Chlorpyridin-2-yl)-N'-[4-(2-oxo-1,3-oxazolidin-3-yl)phenyl]pyrazin-2,3-dicarboxamid

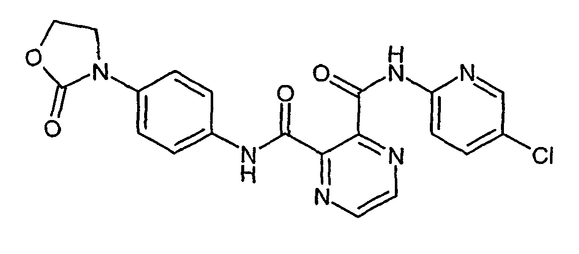

### Stufe a): N-{4-[(2-{[tert.-Butyl(dimethyl)silyl]oxy}ethyl)amino]phenyl}-N'-(5-chlorpyridin-2-yl)pyrazin-2,3-dicarboxamid

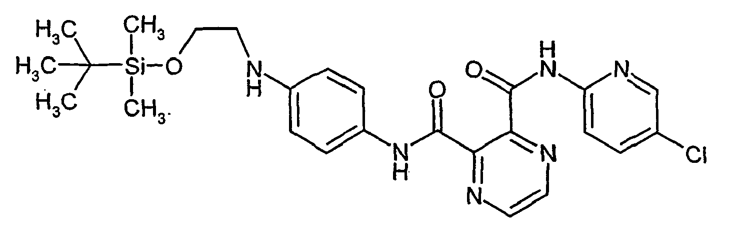

Nach der Allgemeinen Methode 2 werden 104.5 g (0.38 mol) der Verbindung aus Beispiel 12A und 100.0 g (0.38 mol) der Verbindung aus Beispiel 20A umgesetzt.
Ausbeute: 101.3 g (51% d. Th.)
LC-MS (Methode 3): Rₜ = 2.96 min;
MS (ESIpos): m/z = 527 [M+H]⁺;
¹H-NMR (400 MHz, DMSO-d₆): δ = 11.07 (s, 1H), 10.37 (s, 1H), 8.85 (s, 2H), 8.38 (s, 1H), 8.21 (d, 1H), 7.95 (d, 1H), 7.45 (d, 2H), 6.53 (d, 2H), 5.40 (t, NH), 3.67 (t, 2H), 3.10 (dt, 2H), 0.83 (s, 9H), 0.00 (s, 6H).

### Stufe b): N-(5-Chlorpyridin-2-yl)-N'{4-[(2-hydroxyethyl)amino]phenyl}-pyrazin-2,3-dicarboxamid

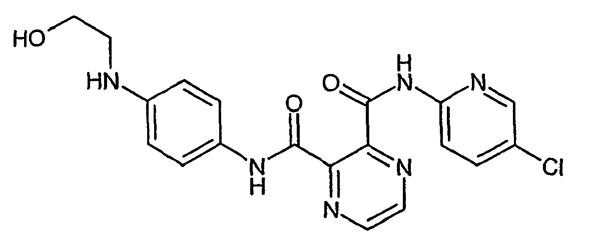

10.0 g (19.0 mmol) *N*-{4-[(2-([*tert.*-Butyl(dimethyl)silyl]oxy}ethyl)amino]phenyl}-*N'*-(5-chlorpyridin-2-yl)pyrazin-2,3-dicarboxamid werden in 50 ml THF gelöst und bei 0°C mit 37.9 ml (9.9 g, 37.9 mmol) Tetra-*n*-butylammoniumfluorid versetzt. Man lässt die Reaktionslösung auf Raumtemperatur aufwärmen und rührt 1 h bei dieser Temperatur. Das Lösungsmittel wird im Vakuum entfernt und der Rückstand mit Dichlormethan und Wasser versetzt. Die organische Phase wird abgetrennt, über Natriumsulfat getrocknet, filtriert und im Vakuum eingeengt. Der erhaltene Feststoff wird in Diethylether verrührt, abfiltriert und im Vakuum getrocknet.
Ausbeute: 4.9 g (63% d. Th.)
LC-MS (Methode 2): Rₜ = 1.54 min;
MS (ESIpos): m/z = 413 [M+H]⁺.

### Stufe c): N-(5-Chlorpyridin-2-yl)-N'-[4-(2-oxo-1,3-oxazolidin-3-yl)phenyl]pyrazin-2,3-dicarboxamid

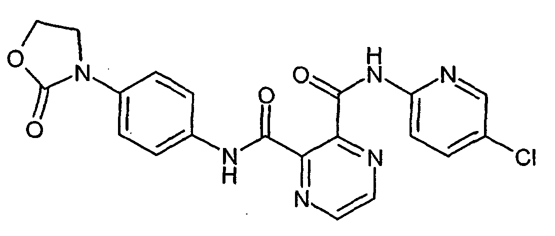

500 mg (1.2 mmol) *N*-(5-Chlorpyridin-2-yl)-*N'*-{4-[(2-hydroxyethyl)amino]phenyl}pyrazin-2,3-dicarboxamid in 20 ml THF werden mit 295 mg (1.8 mmol) 1,1'-Carbonyldiimidazol versetzt. Anschließend werden 74 mg (0.6 mmol) *N*,*N*-4-Dimethylaminopyridin hinzugefügt und das Reaktionsgemisch 16 h bei 80°C gerührt. Das Lösungsmittel wird im Vakuum entfernt und der Rückstand mittels präparativer HPLC gereinigt.
Ausbeute: 100 mg (19% d. Th.)
HPLC (Methode 7): Rₜ = 3.89 min;
MS (ESIpos): m/z = 439 [M+H]⁺;
¹H-NMR (400 MHz, DMSO-d₆): δ = 11.12 (s, 1H), 10.80 (s, 1H), 8.96 (s, 2H), 8.42 (s, 1H), 8.24 (d, 1H), 7.99 (d, 1H), 7.81 (d, 2H), 7.53 (dd, 2H), 4.43 (t, 2H), 4.05 (t, 2H).

### Beispiel 8

### N-(4-Chlorphenyl)-N'-[4-(2-oxoimidazolidin-1-yl)phenyl]pyrazin-2,3-dicarboxamid

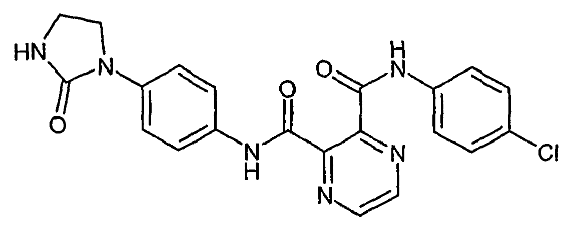

Nach der Allgemeinen Methode 1 wird die Titelverbindung ausgehend von Beispiel 17A dargestellt.
Ausbeute: 34.9 mg (40% d. Th.)
LC-MS (Methode 3): Rₜ= 1.92 min;
MS (ESIpos): m/z = 437 [M+M]⁺.

### Beispiel 9

### N-(5-Chlorpyridin-2-yl)-N'-[2-fluor-4-(3-hydroxy-2-oxopiperidin-1-yl)phenyl]pyrazin-2,3-dicarboxamid

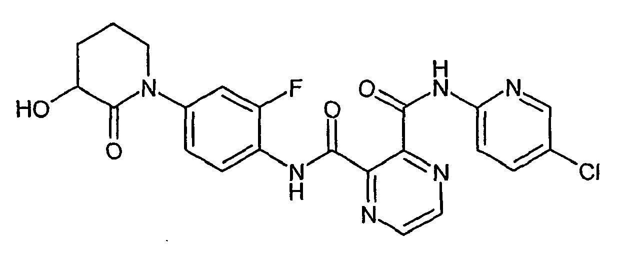

Nach der Allgemeinen Methode 2 werden 2.57 g (11.46 mol) der Verbindung aus Beispiel 4A und 3.19 g (11.46 mol) der Verbindung aus Beispiel 12A umgesetzt.
Ausbeute: 3.23 g (58% d. Th.)
LC-MS (Methode 2): Rₜ = 1.94 min;
MS (ESIpos): m/z = 485 [M+H]⁺;
¹H-NMR (400 MHz, DMSO-d₆): δ = 11.06 (s, 1H), 10.54 (s, 1H), 8.96 (s, 2H), 8.41 (s, 1H), 8.23 (d, 1H), 7.99 (dd, 1H), 7.86 (t, 1H), 7.41 (dd, 1H), 7.18 (d, 1H), 5.30 (d, 1H), 4.12-4.02 (m, 1H), 3.74-3.64 (m, 1H), 3.61-3.50 (m, 1H), 2.17-2.04 (m, 1H), 2.02-1.81 (m, 2H), 1.81-1.69 (m, 1H).

Die Enantiomeren werden durch Chromatographie an chiraler Phase getrennt [Säule: KBD 5326, 640 x 40 mm, basierend auf dem Selektor Poly(*N-*methacryloyl-L-leucin-dicyclopropylmethylamid); Injektionsvolumen: 10 ml; Eluent A: *iso*-Hexan, Eluent B: Ethylacetat; Gradient: 0 min 70% B → 40 min 100% B → 45 min 70% B; Flussrate: 50 ml/min; Säulentemperatur: 24°C; Wellenlänge: 280 nm].

### Enantiomer 1:

>99% ee, Rₜ = 3.58 min [Säule: *ent*-KBD 5326, 250 x 4.6 mm; Eluent: Ethylacetat; Flussrate: 1.0 ml/min; Säulentemperatur: 24°C; Wellenlänge: 270 nm].

### Enantiomer 2:

98% ee, Rₜ = 4.38 min [Säule: KBD 5326, 250 x 4.6 mm; Eluent: Ethylacetat; Flussrate: 1.0 ml/min; Säulentemperatur: 24°C; Wellenlänge: 270 nm].

### Beispiel 10

### N-[4-(3-Amino-2-oxopiperidin-1-yl)phenyl]-N'-(4-chlorphenyl)pyrazin-2,3-dicarboxamid-Hydrochlorid

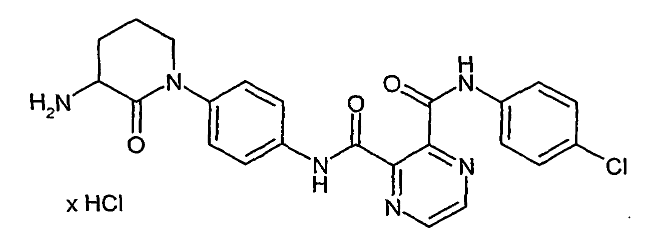

### Stufe a): tert.-Butyl-[1-(4-{[(3-{[(4-chlorphenyl)amino]carbonyl}pyrazin-2-yl)carbonyl]-amino} phenyl)-2-oxopiperidin-3-yl]carbamat

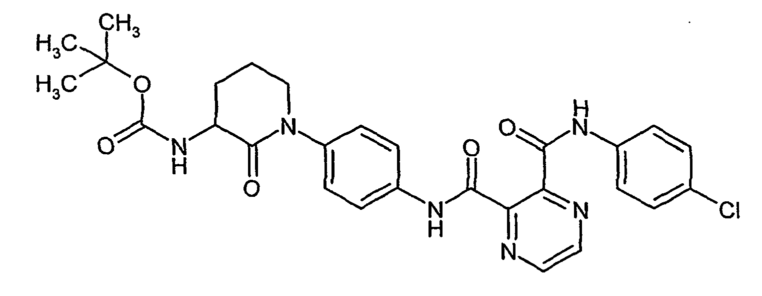

Nach der Allgemeinen Methode 3 werden 560 mg (1.83 mmol) der Verbindung aus Beispiel 5A und 560 mg (2.02 mmol) der Verbindung aus Beispiel 14A umgesetzt.
Ausbeute: 0.12 g (12% d. Th.)
LC-MS (Methode 1): Rₜ = 2.13 min;
MS (ESIpos): m/z = 565 [M+H]⁺;
¹H-NMR (400 MHz, DMSO-d₆): δ= 10.88 (s, 1H), 10.78 (s, 1H), 8.95 (s, 2H), 7.79 (d, 2H), 7.74 (d, 2H), 7.42 (d, 2H), 7.25 (d, 2H), 6.99 (d, 1H), 4.16-4.03 (m, 1H), 3.69-3.52 (m, 2H), 2.09-1.76 (m, 4H), 1.39 (s, 9H).

### Stufe b): N-[4-(3-Amino-2-oxopiperidin-1-yl)phenyl]-N'-(4-chlorphenyl)pyrazin-2,3-dicarboxamid-Hydrochlorid

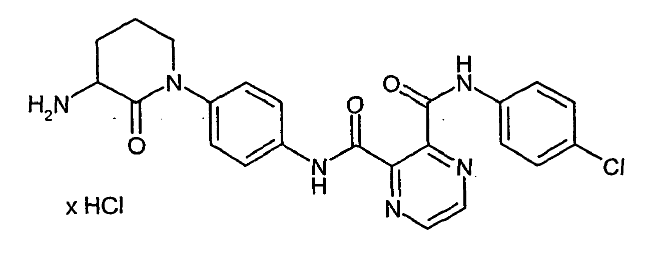

Eine Lösung von 100 mg (0.18 mmol) tert.-Butyl-[1-(4-{[(3-{[(4-chlorphenyl)amino]carbonyl}-pyrazin-2-yl)carbonyl]amino}phenyl)-2-oxopiperidin-3-yl]carbamat in 5 ml Dioxan wird bei RT mit 2 ml einer 4 N Lösung von Chlorwasserstoff in Dioxan versetzt. Die Reaktionssuspension wird über Nacht bei RT gerührt. Das Lösungsmittel wird im Vakuum entfernt und der Rückstand in Diethylether verrührt, abgesaugt und im Hochvakuum getrocknet.
Ausbeute: 76 mg (92% d. Th.)
LC-MS (Methode 1): Rₜ = 1.21 min;
MS (ESIpos): m/z = 465 [M+H]⁺;
¹H-NMR (400 MHz, DMSO-d₆): δ = 10.92 (s, 1H), 10.85 (s, 1H), 8.97 (s, 2H), 8.42-8.28 (m, 3H), 7.8 (t, 4H), 7.41 (d, 2H), 7.3 (d, 2H), 4.13-4.0 (m, 1H), 3.78-3.53 (m, 2H), 2.21-2.3 (m, 1H), 1.98-2.31 (m, 1H), 1.98-1.84 (m, 1H).

Nach der Allgemeinen Methode 1 wird die folgende Verbindung ausgehend von Beispiel 18A dargestellt:

### Beispiel 11

### N-(4-Chlorphenyl)-N'-[4-(3-oxomorpholin-4-yl)phenyl]pyrazin-2,3-dicarboxamid

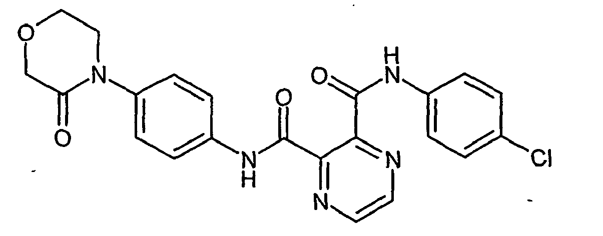

Ausbeute: 9.7 mg (22% d. Th.)
LC-MS (Methode 1): Rₜ = 1.71min;
MS (ESIpos): m/z = 452 [M+H]⁺;
¹H-NMR (200 MHz, DMSO-d₆): δ = 10.9 (s, 1H), 10.83 (s, 1H), 8.97 (s, 2H), 7.9-7.7 (m, 4H), 7.5-7.3 (m, 4H), 4.21 (s, 2H), 3.98 (t, 2H), 3.61 (t, 2H).

### Beispiel 12

### N-(5-Chlorpyridin-2-yl)-N'-[4-(3-oxomorpholin-4-yl)phenyl]pyrazin-2,3-dicarboxamid

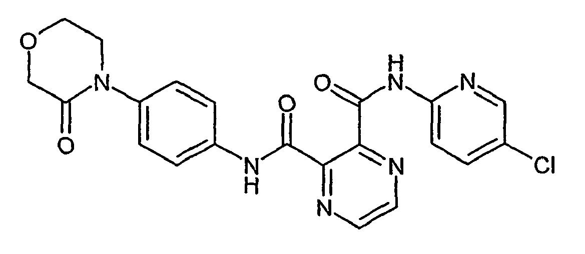

Nach der Allgemeinen Methode 2 werden 10.0 g (34.1 mmol) der Verbindung aus Beispiel 12A und 6.6 g (34.1 mmol) der Verbindung aus Beispiel 6A umgesetzt.
Ausbeute: 12.3 g (79% d. Th.)
LC-MS (Methode 4): Rₜ = 1.84 min;
MS (ESIpos): m/z = 453 [M+H]⁺;
¹H-NMR (300 MHz, DMSO-d₆): δ = 11.06 (s, 1H), 10.82 (s, 1H), 8.93 (s, 2H), 8.41 (s, 1H), 8.23 (d, 1H), 8.02-7.92 (dd, 1H), 7.8 (d, 2H), 7.36 (d, 2H), 4.19 (s, 2H), 3.97 (t, 2H), 3.71 (t, 2H).

### Beispiel 13

### N-(5-Chlorpyridin-2-yl)-N'-[2-fluor-4-(3-oxomorpholin-4-yl)phenyl]pyrazin-2,3-dicarboxamid

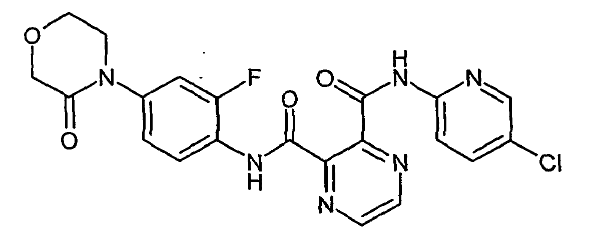

Nach der Allgemeinen Methode 3 werden 500 mg (1.79 mmol) der Verbindung aus Beispiel 7A und 415 mg (1.97 mmol) der Verbindung aus Beispiel 12A umgesetzt.
Ausbeute: 96 mg (11% d. Th.)
LC-MS (Methode 2): Rₜ = 1.90 min;
MS (ESIpos): m/z = 471 [M+H]⁺;
¹H-NMR (400 MHz, DMSO-d₆): δ = 11.05 (s, 1H), 10.57 (s, 1H), 8.98 (s, 2H), 8.42 (s, 1H), 8.25 (d, 1H), 7.98 (d, 1H), 7.87 (t, 1H), 7.48 (d, 1H), 7.29 (d, 1H), 4.22 (s, 2H), 3.98 (t, 2H), 3.77 (t, 2H).

### Beispiel 14

### N-(6-Chlorpyridin-3-yl)-N',[4-(3-oxomorpholin-4-yl)phenyl]pyrazin-2,3-dicarboxamid

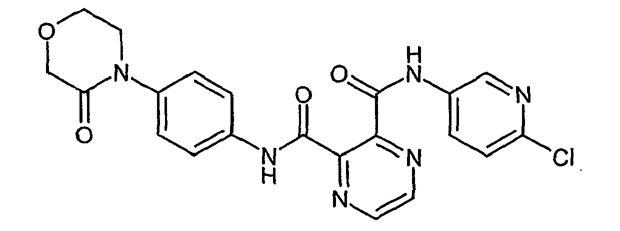

Ausbeute: 5 mg (11% d. Th.)
LC-MS (Methode 3): Rₜ = 1.6 min;
MS (ESIpos): m/z = 453 [M+H]⁺.

### Beispiel 15

### N-(2-Chlorphenyl)-N'-[4-(3-oxomorpholin-4-yl)phenyl]pyrazin-2,3-dicarboxamid

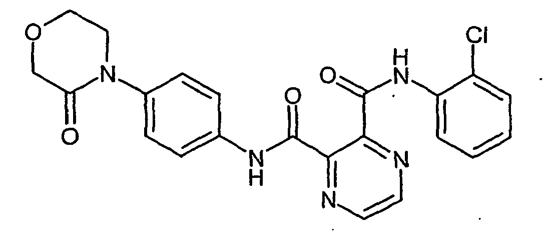

Ausbeute: 0.2 mg (0.2% d. Th.)
LC-MS (Methode 3): Rₜ = 1.92 min;
MS (ESIpos): m/z = 452 [M+H]⁺.

### Beispiel 16

### N-(3,5-Dichlorphenyl)-N'-[4-(3-oxomorpholin-4-yl)phenyl]pyrazin-2,3-dicarboxamid

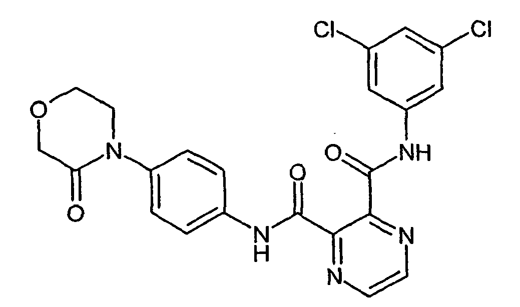

Ausbeute: 6 mg (12% d. Th.)
LC-MS (Methode 3): Rₜ = 2.13 min;
MS (ESIpos): m/z = 486 [M+H]⁺.

### Beispiel 17

### N-(4-Fluorphenyl)-N'-[4-(3-oxomorpholin-4-yl)phenyl]pyrazin-2,3-dicarboxamid

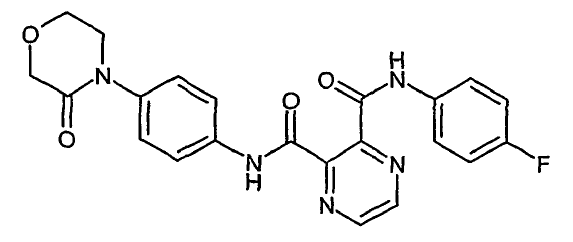

Ausbeute: 28 mg (64% d. Th.)
LC-MS (Methode 4): Rₜ = 1.95 min;
MS (ESIpos)- m/z = 436 [M+H]⁺.

### Beispiel 18

### N-(4-Methylphenyl)-N'-[4-(3-oxomorpholin-4-yl)phenyl]pyrazin-2,3-dicarboxamid

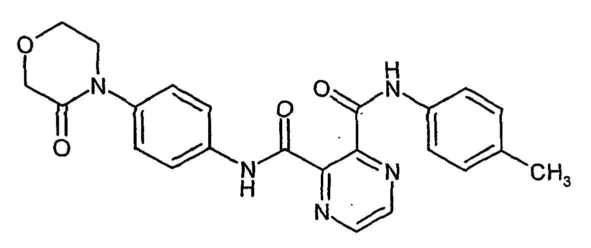

Ausbeute: 24 mg (56% d. Th.)
LC-MS (Methode 3): Rₜ = 1.82 min;
MS (ESIpos): m/z = 432 [M+H]⁺.

### Beispiel 19

### N-[4,(3-Oxomorpholin-4-yl)phenyl]-N'-phenylpyrazin-2,3-dicarboxamid

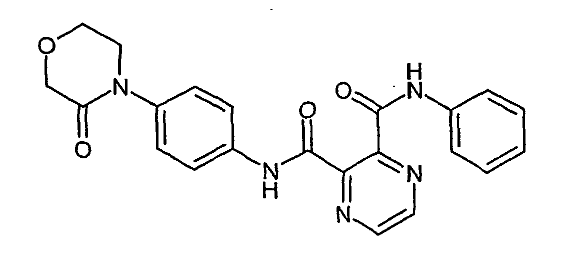

Ausbeute: 31 mg (74% d. Th.)
LC-MS (Methode 2): Rₜ = 1.65 min;
MS (ESIpos): m/z = 418 [M+H]⁺.

### Beispiel 20

### N-(4-Ethinylphenyl)-N'-[4-(3-oxomorpholin-4-yl)phenyl]pyrazin-2,3-dicarboxamid

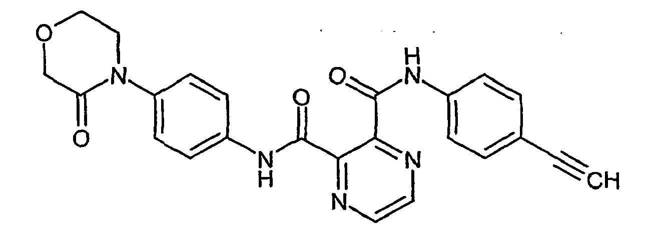

Nach der Allgemeinen Methode 2 werden 72 mg (0.37 mmol) der Verbindung aus Beispiel 6A und 100 mg (0.37 mmol) der Verbindung aus Beispiel 13A umgesetzt.
Ausbeute: 19 mg (11% d. Th.)
LC-MS (Methode 1): Rₜ = 1.61 min;
MS (ESIpos): m/z = 442 [M+H]⁺;
¹H-NMR (300 MHz, DMSO-d₆): δ = 10.92 (s, 1H), 10.84 (s, 1H), 8.96 (s, 2H), 7.78 (dd, 4H), 7.48 (d, 2H), 7.38 (d, 2H), 4.20 (s, 2H), 4.13 (s, 1H), 3.97 (dd, 2H), 3.72 (dd, 2H).

### Beispiel 21

### N-(4-Chlorphenyl)-N'-{4-[2-(2-hydroxyethyl)-3-oxomorpholin-4-yl]phenyl}pyrazin-2,3-dicarboxamid

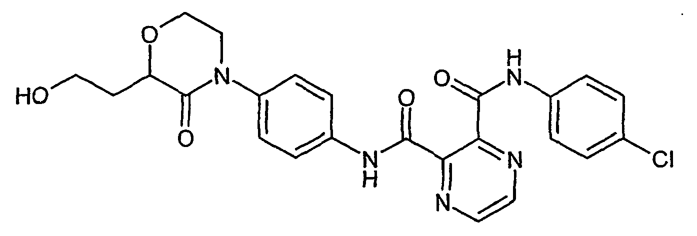

### Stufe a): N-{4[2-(2-{[tert.-Butyl(dimethyl)silyl]oxy}ethyl)-3-oxomorpholin-4-yl]phenyl}-N'-(4-chlorphenyl)pyrazin-2,3-dicarboxamid

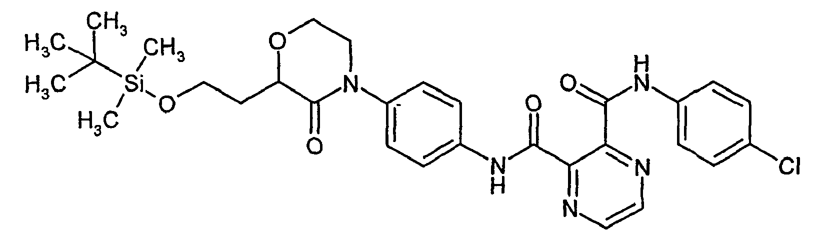

Eine Lösung von 150 mg (0.43 mmol) 4-(4-Aminophenyl)-2-(2-{[tert.-butyl(dimethyl)silyl]oxy}-ethyl)morpholin-3-on (Beispiel 8A) in 1.5 ml DMF wird bei RT unter Argon mit 64 mg (0.43 mmol, 1.0 eq.) 2,3-Pyrazindicarbonsäureanhydrid versetzt und 0.5 h gerührt. Anschließend werden 50 µl (0.39 mmol, 0.9 eq.) Triethylamin und 60 µl (0.47 mmol, 1.1 eq.) 2,2-Dimethylpropanoylchlorid zugegeben. Das Reaktionsgemisch wird 1 h bei RT gerührt, dann mit 55 mg (0.43 mmol, 1.0 eq.) 4-Chloranilin versetzt und weitere 4 h bei RT gerührt. Nach Zugabe von Wasser und gesättigter wässriger Natriumhydrogencarbonat-Lösung und Phasentrennung wird die wässrige Phase mit Ethylacetat extrahiert. Die vereinigten organischen Phasen werden über Natriumsulfat getrocknet, filtriert und im Vakuum eingeengt. Das Rohprodukt wird mittels präparativer HPLC gereinigt.
Ausbeute: 66 mg (70% Reinheit, 70% d. Th.)
LC-MS (Methode 1): Rₜ = 2.80 min;
MS (ESIpos): m/z = 611 [M+H]⁺.

### Stufe b): N-(4-Chlorphenyl)-N'-{4-[2-(2-hydroxyethyl)-3-oxomorpholin-4-yl]phenyl}-pyrazin-2,3-dicarboxamid

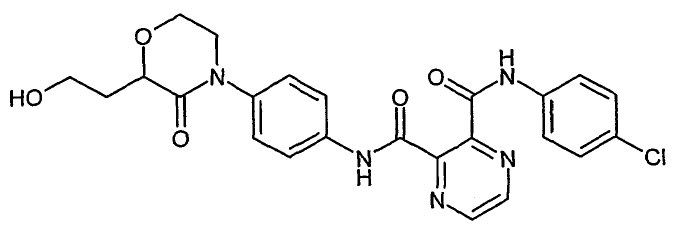

Eine Lösung von 66 mg (70%-ig, 0.08 mmol) *N*-{4-[2-(2-{[*tert.*-Butyl(dimethyl)silyl]oxy}ethyl)-3-oxomorpholin-4-yl]phenyl}-*N'*-(4-chlorphenyl)pyrazin-2,3-dicarboxamid in 1 ml THF wird bei RT mit 160 µl (0.16 mmol, 2.0 eq.) Tetra-*n*-butylammoniumfluorid versetzt und über Nacht bei RT gerührt. Das Reaktionsgemisch wird mit Wasser und Dichlormethan verdünnt, und nach Phasentrennung wird die wässrige Phase mit Dichlormethan extrahiert. Die vereinigten organischen Phasen werden über Magnesiumsulfat getrocknet, filtriert und im Vakuum eingeengt.
Ausbeute: 25 mg (97% Reinheit, 64% d. Th.)
LC-MS (Methode 1): Rₜ = 1.63 min;
MS (ESIpos): m/z = 496 [M+H]⁺;
¹H-NMR (400 MHz, CDCl₃): δ = 9.05 (d, 2H), 8.7 (s, 2H), 7.73 (d, 2H), 7.68 (d, 2H), 7.3 (dd, 4H), 4.42 (t, 1H), 4.12-4.2 (m, 1H), 3.98 (d, 2H), 3.89-3.8 (m, 2H), 3.62-3.52 (m, 1H), 2.4 (t, 1H), 2.32-2.08 (m, 2H).

Nach der Allgemeinen Methode 1 werden die folgenden Verbindungen ausgehend von Beispiel 19A dargestellt:

### Beispiel 22

### N-(4-Chlorphenyl)-N'-[4-(3-methyl-2-oxotetrahydropyrimidin-1(2H)-yl)phenyl]pyrazin-2,3-dicarboxamid

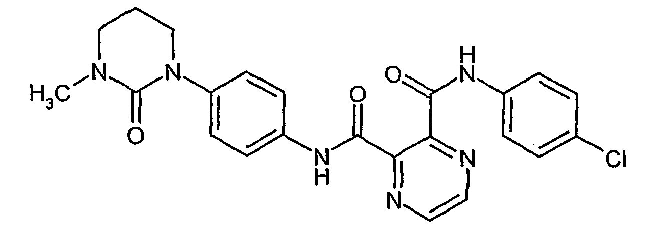

Ausbeute: 17.3 mg (37% d. Th.)
LC-MS (Methode 1): Rₜ = 1.85 min;
MS (ESIpos): m/z = 465 [M+H]⁺;
¹H-NMR (400 MHz, DMSO-d₆): δ = 10.85 (s, 1H), 10.69 (s, 1H), 8.94 (s, 2H), 7.8 (d, 2H), 7.66 (d, 2H), 7.42 (d, 2H), 7.21 (d, 2H), 3.62 (t, 2H), 3.39-3.34 (m, 2H), 2.85 (s, 3H), 1.98-2.09 (m, 2H).

### Beispiel 23

### N-(5-Chlorpyridin-2-yl)-N'-[4-(3-methyl-2-oxotetrahydropyrimidin-1(2H)-yl)phenyl]pyrazin-2,3-dicarboxamid

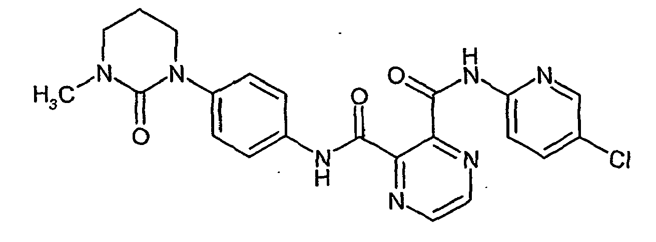

Ausbeute: 2.3 mg (5% d. Th.)
LC-MS (Methode 3): Rₜ = 1.93 min;
MS (ESIpos): m/z = 466 [M+H]⁺.

### Beispiel 24

### N-(6-Chlorpyridin-3-yl)-N'-[4-(3-methyl-2-oxotetrahydropyrimidin-1(2H)-yl)phenyl]pyrazin-2,3-dicarboxamid

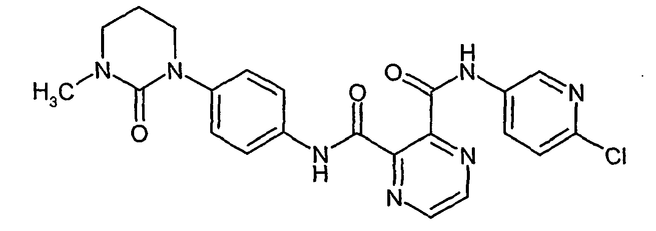

Ausbeute: 9 mg (19% d. Th.)
LC-MS (Methode 3): Rₜ = 1.74 min;
MS (ESIpos): m/z = 466 [M+H]⁺.

### Beispiel 25

### N-(4-Fluorphenyl)-N'[4-(3-methyl-2-oxotetrahydropyrimidin-1(2H)-yl)phenyl]pyrazin-2,3-dicarboxamid

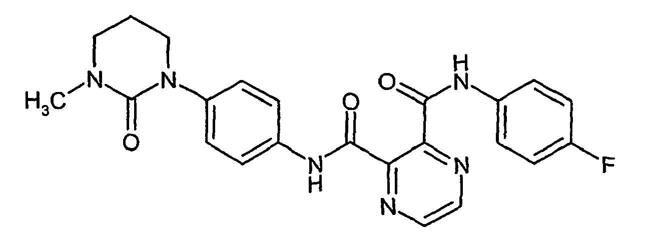

Ausbeute: 30 mg (67% d. Th.)
LC-MS (Methode 4): Rₜ = 2.07 min;
MS (ESIpos): m/z = 448 [M+H]⁺.

### Beispiel 26

### N-[4-(3-Methyl-2-oxotetrahydropyrimidin-1(2H)-yl)phenyl]-N'-(4-methylphenyl)pyrazin-2,3-dicarboxamid

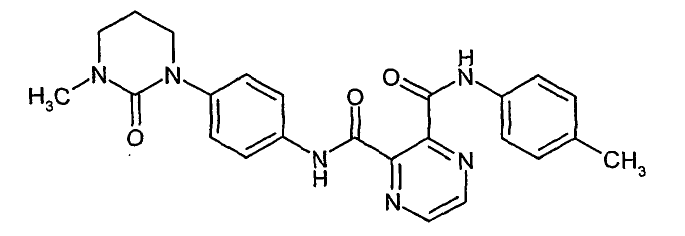

Ausbeute: 22 mg (49% d. Th.)
LC-MS (Methode 3): Rₜ = 1.94 min;
MS (ESIpos): m/z = 445 [M+H]⁺.

### Beispiel 27

### N-(5-Chlorpyridin-2-yl)-N'-{4-[3-(2-hydroxyethyl)-2-oxotetrahydropyrimidin- 1(2H)-yl]phenyl}-pyrazin-2,3-dicarboxamid

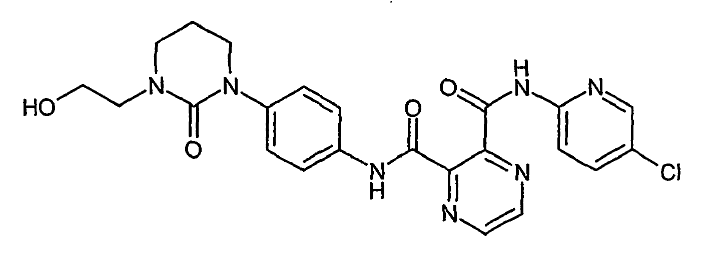

Eine Lösung aus 169 mg (0.61 mmol, 1.1 eq.) der Verbindung aus Beispiel 12A in 2 ml Dichlormethan und 2 ml DMF wird bei 0°C mit 231 mg (0.61 mmol, 1.1 eq.) O-(7-Azabenzotriazol-1-yl)-*N,N,N',N*'-tetramethyluronium-hexafluorophosphat (HATU), 130 mg (0.55 mmol) der Verbindung aus Beispiel 10A und 340 µl (1.93 mmol, 3.5 eq.) *N,N*-Diisopropylethylamin versetzt. Das Reaktionsgemisch wird 30 min bei 0°C gerührt, anschließend auf RT kommen gelassen und über Nacht gerührt. Das Reaktionsgemisch wird im Vakuum eingeengt und der Rückstand mittels RP-HPLC gereinigt.
Ausbeute: 17 mg (6% d. Th.)
LC (Methode 3): Rₜ = 1.74 min;
MS (ESIpos): m/z = 496 [M+H]⁺;
¹H-NMR (400 MHz, DMSO-d₆)- δ = 11.11 (s, 1H), 10.75 (s, 1H), 8.92 (s, 2H), 8.42 (s, 1H), 8.25 (d, 1H), 7.99 (dd, 1H), 7.40 (d, 2H), 7.22 (d, 2H), 4.68 (t, 1H), 3.61 (t, 2H), 3.52 (m, 2H), 3.43 (t, 2H), 2.01 (quintett, 2H).

### Beispiel 28

### N-(5-Chlorpyridin-2-yl)-N'-{4-[2-oxo-3-(2-pyrrolidin-1-ylethyl)tetrahydropyrimidin-1(2H)-yl]-phenyl} pyrazin-2,3-dicarboxamid

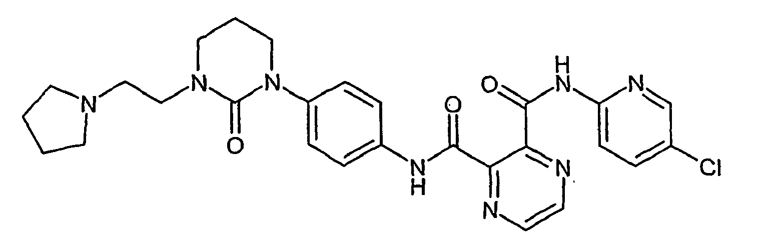

Eine Suspension von 140 mg (0.28 mmol) der Verbindung aus Beispiel 27 in 10 ml THF und 5 ml DMF wird bei -78°C mit 60 µl (0.34 mmol, 1.2 eq.) Trifluormethansulfonsäureanhydrid und 0.10 ml (0.85 mmol, 3 eq.) 2,6-Dimethylpyridin versetzt. Das Reaktionsgemisch wird 2 h bei -78°C gerührt, anschließend langsam bis -5°C kommen gelassen und dann mit 0.24 ml (2.82 mmol, 10 eq.) Pyrrolidin versetzt. Die Reaktionslösung wird langsam auf RT erwärmt und über Nacht bei RT gerührt. Das Reaktionsgemisch wird im Vakuum eingeengt und der Rückstand mittels RP-HPLC gereinigt.
Ausbeute: 25 mg (15% d. Th.)
LC (Methode 3): Rₜ = 1.54 min;
MS (ESIpos): m/z = 549 [M+H]⁺;
¹H-NMR (300 MHz, DMSO-d₆): δ = 11.20-11.03 (br. s, 1H), 10.77 (s, 1H), 8.92 (s, 2H), 8.40 (s, 1H), 8.25 (d, 1H), 8.00 (dd, 1H), 7.70 (d, 2H), 7.23 (d, 2H), 3.65-3.58 (m, 2H), 3.14-3.05 (m, 4H), 2.77-2.60 (m, 6H), 2.07-1.96 (m, 2H), 1.87-1.79 (m, 4H), 1.77-1.68 (m, 4H).

### Beispiel 29

### N-(5-Chlorpyridin-2-yl)-N'-{2-fluor-4-[3-(hydroxymethyl)-2-oxopyridin-1(2H)-yl]phenyl}pyrazin-2,3-dicarboxamid

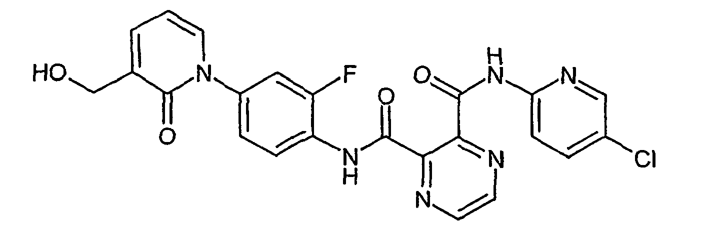

Nach der Allgemeinen Methode 2 werden 2.00 g (8.54 mmol) der Verbindung aus Beispiel 11A und 2.38 g (8.54 mmol) der Verbindung aus Beispiel 12A umgesetzt.
Ausbeute: 2.55 g (60% d. Th.)
LC-MS (Methode 1): Rₜ = 1.58 min;
MS (ESIpos): m/z = 495 [M+M]⁺;
¹H-NMR (300 MHz, DMSO-d₆): δ = 11.08 (s, 1H), 10.69 (s, 1H), 8.98 (s, 2H), 8.43 (s, 1H), 8.25 (d, 1H), 8.08-7.94 (m, 2H), 7.59 (dd, 1H), 7.51 (dd, 2H), 7.29 (dd, 1H), 6.39 (t, 1H), 5.18 (t, 1H), 4.34 (d, 2H).

### Beispiel 30

### N-(5-Chlorpyridin-2-yl)-N'-{4-[3-[(cyclopropylamino)methyl]-2-oxopyridin-1(2H)-yl]-2-fluorphenyl}pyrazin-2,3-dicarboxamid-Trifluoracetat

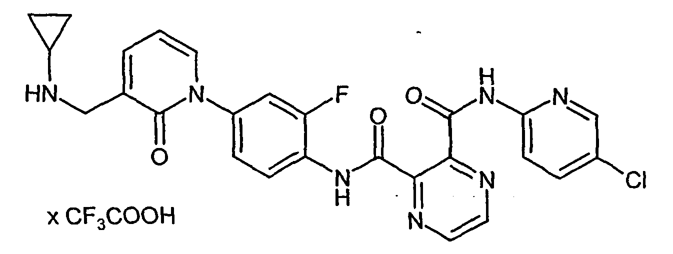

### Stufe a): N-(5-Chlorpyridin-2-yl)-N'-[2-fluor-4-(3-formyl-2-oxopyridin-1(2H)-yl)phenyl]-pyrazin-2,3-dicarboxamid

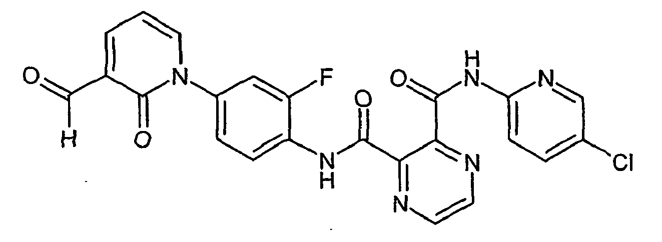

Eine Suspension von 2.27 g (4.58 mmol) der Verbindung aus Beispiel 29 in 60 ml Dichlormethan wird unter Argon bei RT mit 2.14 g (5.04 mmol, 1.1 eq.) Dess-Martin-Periodinan versetzt. Das Reaktionsgemisch wird 1 h bei RT gerührt, wobei nach wenigen Minuten aus der Suspension eine Lösung wird und anschließend ein Niederschlag gebildet wird. Der Niederschlag wird abfiltriert, getrocknet und ohne weitere Reinigung in der nächsten Stufe umgesetzt.
Ausbeute: 2.21 g

### Stufe b): N-(5-Chlorpyridin-2-yl)-N'-{4-[3-[(cyclopropylamino)methyl]-2-oxopyridin-1(2H)-yl]-2-fluorphenyl}pyrazin-2,3-dicarboxamid-Trifluoracetat

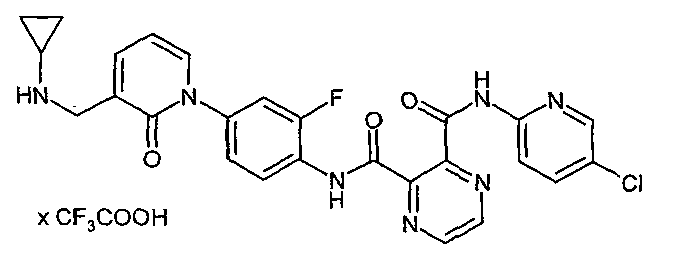

Eine Suspension von 100 mg (0.20 mmol) *N*'(5-Chlorpyridin-2-yl)-*N'*-[2-fluor-4-(3-formyl-2-oxo-pyridin-1(2*H*)-yl)phenyl]pyrazin-2,3-dicarboxamid in 5 ml Methanol wird unter Argon bei RT mit 70 µl (1.01 mmol, 5 eq.) Cyclopropylamin versetzt und 3 h bei RT gerührt. Anschließend wird das Reaktionsgemisch im Eisbad gekühlt, portionsweise mit 19 mg (0.51 mmol, 2.5 eq.) Natriumborhydrid versetzt und dann über Nacht bei RT gerührt. Der Rückstand wird mit gesättigter wässriger Natriumchlorid-Lösung und Dichlormethan versetzt, und nach Phasentrennung wird die wässrige Phase mit Dichlormethan extrahiert. Die vereinigten organischen Phasen werden über Magnesiumsulfat getrocknet, filtriert und im Vakuum eingeengt. Das Rohprodukt wird mittels präparativer HPLC gereinigt (Kromasil 100 C18 5 µm, Eluent: Wasser/Acetonitril/1%-ige Trifluoressigsäure 48:40:12).
Ausbeute: 4.8 mg (4% d. Th.)
LC-MS (Methode 1): Rₜ = 1.26 min;
MS (ESIpos): m/z = 534 [M+H]⁺;
¹H-NMR (300 MHz, DMSO-d₆): δ = 11.08 (s, 1H), 10.70 (s, 1H), 8.98 (d, 2H), 8.75 (br. s, 2H), 8.41 (s, 1H), 8.22 (d, 1H), 8.08 (t, 1H), 7.99 (dd, 1H), 7.86 (dd, 1H), 7.78 (dd, 1H), 7.55 (dd, 1H), 7.32 (dd, 1H), 6.49 (t, 1H), 4.10 (s, 2H), 2.78-2.69 (m, 1H), 0.84-0.75 (m, 4H).

### Beispiel 31

### N-(4-Cyanophenyl)-N'-[4-(3-oxomorpholin-4-yl)phenyl]pyrazin-2,3-dicarboxamid

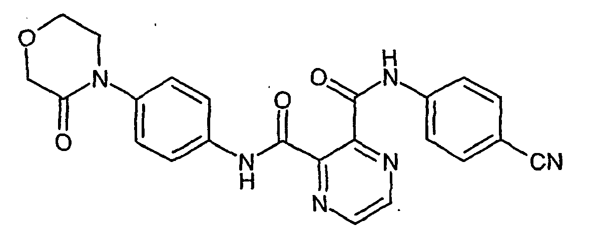

Nach der Allgemeinen Methode 2 werden 143 mg (0.75 mmol) der Verbindung aus Beispiel 6A und 200 mg (0.75 mmol) der Verbindung aus Beispiel 21A umgesetzt.
Ausbeute: 24 mg (7% d. Th.)
LC-MS (Methode 3): Rₜ = 1.63 min;
MS (ESIpos): m/z = 443 [M+H]⁺;
¹H-NMR (400 MHz, DMSO-d₆): δ = 11.18 (s, 1H), 10.87 (s, 1H), 8.98 (s, 2H), 7.96 (d, 2H), 7.83 (d, 2H), 7.79 (d, 2H), 7.38 (d, 2H), 4.20 (s, 2H), 3.98 (t, 2H), 3.72 (t, 2H).

### Beispiel 32

### N-[2-(2-Aminoethoxy)-4-(3-oxomorpholin-4-yl)phenyl]-N'-(5-chlorpyridin-2-yl)pyrazin-2,3-dicarboxamid-Trifluoracetat

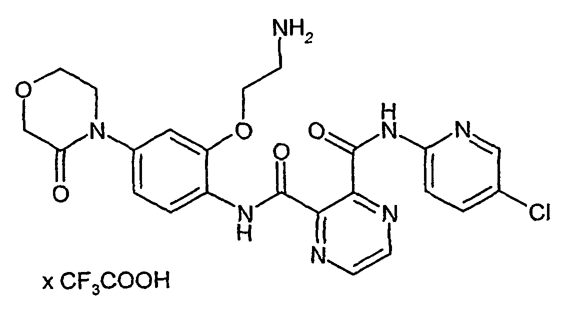

### Stufe a): N-(5-Chlorpyridin-2-yl)-N'-[2-[2-(1,3-dioxo-1,3-dihydro-2H-isoindol-2-yl)ethoxy]-4-(3-oxomorpholin-4-yl)phenyl]pyrazin-2,3-dicarboxamid

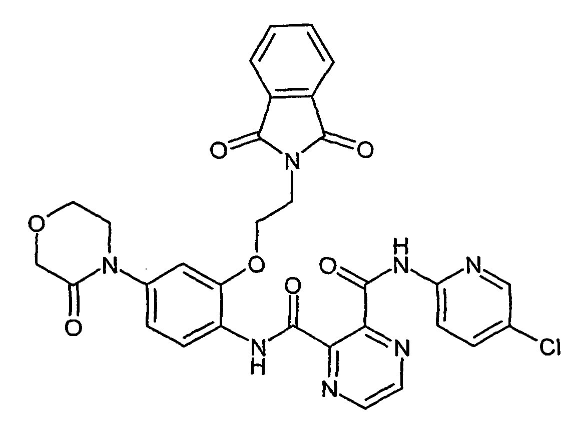

Nach der Allgemeinen Methode 2 werden 146 mg (0.52 mmol) der Verbindung aus Beispiel 12A und 200 mg (0.52 mmol, 1.0 eq.) der Verbindung aus Beispiel 22A umgesetzt. Es wird 16 Stunden bei Raumtemperatur gerührt und dann erneut 563 mg (2.02 mmol, 3.9 eq.) der Verbindung aus Beispiel 12A zugegeben. Nach weiteren 16 Stunden Rühren werden 20 ml Wasser zugesetzt und die organische Phase abgetrennt. Diese wird mit 20 ml einer gesättigten wässrigen Natriumhydrogencarbonat-Lösung sowie mit 20 ml Wasser gewaschen. Die organische Phase wird im Vakuum eingeengt und der Rückstand mittels präparativer RP-HPLC gereinigt.
Ausbeute: 127 mg (38% d. Th.)
LC-MS (Methode 3): Rₜ = 2.18 min;
MS (ESIpos): m/z = 642 [M+H]⁺;
¹H-NMR (400 MHz, DMSO-d₆): δ = 11.10 (s, 1H), 10.03 (s, 1H), 8.93-8.91 (m, 1H), 8.79-8.77 (m, 1H), 8.38-8.36 (m, 1H), 8.23 (d, 1H), 8.20 (d, 1H), 7.96 (d, 1H), 7.82-7.75 (m, 4H), 7.24-7.22 (m, 1H), 6.95 (d, 1H), 4.37 (t, 2H), 4.16 (s, 2H), 4.02 (t, 2H), 3.94 (t, 2H), 3.68 (t, 2H).

### Stufe b): N-[2-(2-Aminoethoxy)-4-(3-oxomorpholin-4-yl)phenyl]-N'-(5-chlorpyridin-2-yl)-pyrazin-2,3-dicarboxamid-Trifluoracetat

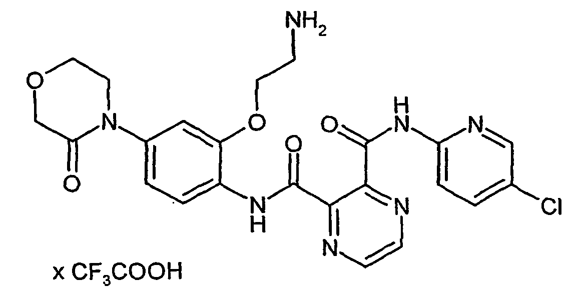

Eine Suspension von 113 mg (0.18 mmol) *N-*(5-Chlorpyridin-2-yl)-*N*'-[2-[2-(1,3-dioxo-1,3-dihydro-2*H*-isoindol-2-yl)ethoxy]-4-(3-oxomorpholin-4-yl)phenyl]pyrazin-2,3-dicarboxamid und 0.205 ml (82.0 mg, 2.64 mmol, 15 eq.) einer 40%-igen wässrigen Methylamin-Lösung in 5 ml Methanol wird 15 Minuten bei 40°C gerührt. Anschließend wird für 25 Minuten bei 60°C gerührt, dann auf Raumtemperatur abgekühlt, eingeengt und der Rückstand mittels präparativer RP-HPLC (Eluent: Wasser/Acetonitril/1%-ige Trifluoressigsäure 56:30:14) gereinigt.
Ausbeute: 61 mg (52% d. Th.)
LC-MS (Methode 3): Rₜ = 1.33 min;
MS (ESIpos): m/z = 512 [M+H-CF₃COOH]⁺;
¹H-NMR (400 MHz, DMSO-d₆): δ = 11.16 (s, 1H), 10.26 (s, 1H), 8.99-8.97 (m, 1H), 8.95-8.93 (m, 1H), 8.44 (s, 1H), 8.23 (d, 1H), 8.07 (d, 1H), 8.01 (br. s, 3H), 8.01-7.98 (m, 1H), 7.24 (s, 1H), 7.03 (d, 1H), 4.29 (t, 2H), 4.21 (s, 2H), 3.99 (t, 2H), 3.74 (t, 2H), 3.29-3.27 (m, 2H).

### B. Bewertung der pharmakologischen Wirksamkeit

Die erfindungsgemäßen Verbindungen wirken insbesondere als selektive Inhibitoren des Blutgerinnungsfaktors Xa und hemmen nicht oder erst bei deutlich höheren Konzentrationen auch andere Serinproteasen wie Plasmin oder Trypsin.

Als "selektiv" werden solche Inhibitoren des Blutgerinnungsfaktors Xa bezeichnet, bei denen die IC₅₀-Werte für die Faktor Xa-Inhibierung gegenüber den IC₅₀-Werten für die Inhibierung anderer Serinproteasen, insbesondere Plasmin und Trypsin, um mindestens das 100-fache kleiner sind, wobei bezüglich der Testmethoden für die Selektivität Bezug genommen wird auf die im folgenden beschriebenen Testmethoden der Beispiele B.a.1) und B.a.2).

Die vorteilhaften pharmakologischen Eigenschaften der erfindungsgemäßen Verbindungen können durch folgende Methoden festgestellt werden:

### a) Testbeschreibungen (in vitro)

### a.1) Messung der Faktor Xa-Hemmung:

Die enzymatische Aktivität von humanem Faktor Xa (FXa) wird über die Umsetzung eines für den FXa-spezifischen chromogenen Substrats gemessen. Dabei spaltet der Faktor Xa aus dem chromogenen Substrat p-Nitroanilin ab. Die Bestimmungen werden wie folgt in Mikrotiterplatten durchgeführt:

Die Prüfsubstanzen werden in unterschiedlichen Konzentrationen in DMSO gelöst und für 10 Minuten mit humanem FXa (0.5 nmol/l gelöst in 50 mmol/l Tris-Puffer [*C*,*C*,*C*-Tris(hydroxymethyl)aminomethan], 150 mmol/l NaCl, 0.1% BSA [bovine serum albumine], pH = 8.3) bei 25°C inkubiert. Als Kontrolle dient reines DMSO. Anschließend wird das chromogene Substrat (150 µmol/l Pefachrome^{®} FXa der Firma Pentapharm) hinzugefügt. Nach 20 Minuten Inkubationsdauer bei 25°C wird die Extinktion bei 405 nm bestimmt. Die Extinktionen der Testansätze mit Prüfsubstanz werden mit den Kontrollansätzen ohne Prüfsubstanz verglichen und daraus die IC₅₀-Werte berechnet.

Repräsentative Wirkdaten aus diesem Test sind in der folgenden Tabelle 1 aufgeführt:

**Tabelle 1**

| **Beispiel Nr.** | **IC₅₀ [nM]** |
|---|---|
| 9 | 4.4 |
| 11 | 0.49 |
| 12 | 0.16 |
| 17 | 16 |
| 18 | 7.5 |
| 20 | 6.3 |
| 30 | 0.44 |
| 32 | 0.3 |

### a.2) Bestimmung der Selektivität;

Zum Nachweis der selektiven FXa-Inhibition werden die Prüfsubstanzen auf ihre Hemmung anderer humaner Serinproteasen wie Trypsin und Plasmin hin untersucht. Zur Bestimmung der enzymatischen Aktivität von Trypsin (500 mU/ml) und Plasmin (3.2 nmol/l) werden diese Enzyme in Tris-Puffer (100 mmol/l, 20 mmol/l CaCl₂, pH = 8.0) gelöst und für 10 Minuten mit Prüfsubstanz oder Lösungsmittel inkubiert. Anschließend wird durch Zugabe der entsprechenden spezifischen chromogenen Substrate (Chromozym Trypsin^{®} und Chromozym Plasmin^{®}; Fa. Roche Diagnostics) die enzymatische Reaktion gestartet und die Extinktion nach 20 Minuten bei 405 nm bestimmt. Alle Bestimmungen werden bei 37°C durchgeführt. Die Extinktionen der Testansätze mit Prüfsubstanz werden mit den Kontrollproben ohne Prüfsubstanz verglichen und daraus die IC₅₀-Werte berechnet.

### a.3) Bestimmung der antikoagulatorischen Wirkung:

Die antikoagulatorische Wirkung der Prüfsubstanzen wird *in vitro* in Human- und Kaninchenplasma bestimmt. Dazu wird Blut unter Verwendung einer 0.11 molaren Natriumcitrat-Lösung als Vorlage in einem Mischungsverhältnis Natriumcitrat/Blut 1:9 abgenommen. Das Blut wird unmittelbar nach der Abnahme gut gemischt und 10 Minuten bei ca. 2500 g zentrifugiert. Der Überstand wird abpipettiert. Die Prothrombinzeit (PT, Synonyme: Thromboplastinzeit, Quick-Test) wird in Gegenwart variierender Konzentrationen an Prüfsubstanz oder dem entsprechenden Lösungsmittel mit einem handelsüblichen Testkit (Hemoliance^{®} RecombiPlastin, Fa. Instrumentation Laboratory) bestimmt. Die Testverbindungen werden 3 Minuten bei 37°C mit dem Plasma inkubiert. Anschließend wird durch Zugabe von Thromboplastin die Gerinnung ausgelöst und der Zeitpunkt des Gerinnungseintritts bestimmt. Es wird die Konzentration an Prüfsubstanz ermittelt, die eine Verdoppelung der Prothrombinzeit bewirkt.

### b) Bestimmung der antithrombotischen Wirkung (in vivo)

### b.1) Arteriovenöses Shunt-Modell (Kaninchen):

Nüchterne Kaninchen (Stamm: Esd: NZW) werden durch intramuskuläre Gabe einer Rompun/Ketavet-Lösung narkotisiert (5 mg/kg bzw. 40 mg/kg). Die Thrombusbildung wird in einem arteriovenösen Shunt in Anlehnung an die von C.N. Berry et al. [Semin. Thromb. Hemost. 1996, 22, 233-241] beschriebene Methode ausgelöst. Dazu werden die linke Vena jugularis und die rechte Arteria carotis freipräpariert. Ein extracorporaler Shunt wird mittels eines 10 cm langen Venenkatheders zwischen den beiden Gefäßen gelegt. Dieser Katheder ist in der Mitte in einen weiteren, 4 cm langen Polyethylenschlauch (PE 160, Becton Dickenson), der zur Erzeugung einer thrombogenen Oberfläche einen aufgerauhten und zu einer Schlinge gelegten Nylonfaden enthält, eingebunden. Der extrakorporale Kreislauf wird 15 Minuten lang aufrechterhalten. Dann wird der Shunt entfernt und der Nylonfaden mit dem Thrombus sofort gewogen. Das Leergewicht des Nylonfadens ist vor Versuchsbeginn ermittelt worden. Die Prüfsubstanzen werden vor Anlegung des extrakorporalen Kreislaufs entweder intravenös über eine Ohrvene oder oral mittels Schlundsonde verabreicht.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

*Einer Einzeldosis von 100 mg der erfindungsgemäßen Verbindung entsprechen 10 ml orale* Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 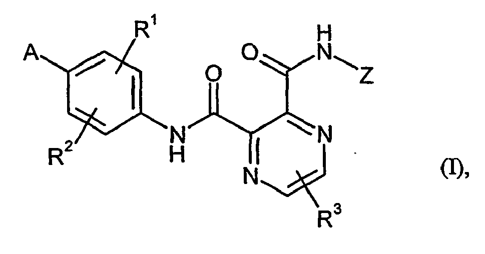 in welcher
A für eine Gruppe der Formel 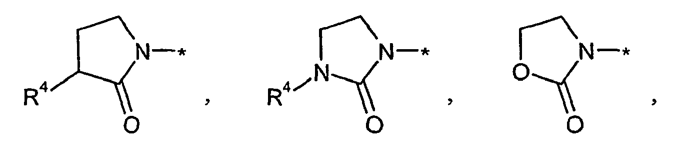 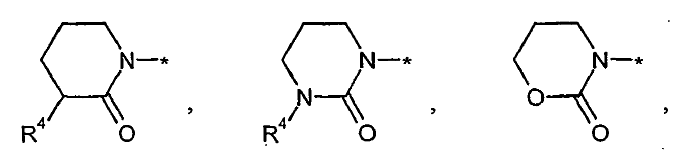 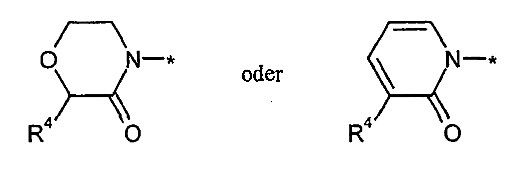 steht, worin
R⁴ Wasserstoff, (C₁-C₆)-Alkyl, Hydroxy, (C₁-C₆)-Alkoxy, Amino, Mono- oder Di-(C₁-C₆)-alkylamino, (C₃-C₇)-Cycloalkylamino, (C₁-C₆)-Alkanoylamino oder (C₁-C₆)-Alkoxycarbonylamino bedeutet, wobei
(C₁-C₆)-Alkyl, (C₁-C₆)-Alkoxy, Mono- und Di-(C₁-C₆)-alkylamino ihrerseits jeweils durch Hydroxy, (C₁-C₄)-Alkoxy, Amino, Mono- oder Di-(C₁-C₄)-alkylamino, (C₃-C₇)-Cycloalkylamino oder einen 4- bis 7-gliedrigen gesättigten, über ein N-Atom gebundenen Heterocyclus, der ein Ringglied aus der Reihe N-R⁵ oder O enthalten kann, worin
R⁵ Wasserstoff oder (C₁-C₄)-Alkyl bedeutet,
substituiert sein können,
und * die Verknüpfungsstelle mit dem Phenylring bedeutet,
Z für Phenyl, Pyridyl, Pyrimidinyl, Pyrazinyl oder Thienyl steht, die jeweils ein- oder zweifach, gleich oder verschieden, durch Substituenten ausgewählt aus der Gruppe von Fluor, Chlor, Cyano, (C₁-C₄)-Alkyl, welches seinerseits durch Amino substituiert sein kann, Ethinyl und Amino substituiert sein können,
R¹ und R² gleich oder verschieden sind und unabhängig voneinander für Wasserstoff, Fluor, Chlor, Cyano, (C₁-C₃)-Alkyl, Cyclopropyl, Trifluormethyl, Hydroxy, (C₁-C₃)-Alkoxy, Trifluormethoxy oder Amino stehen, wobei
(C₁-C₃)-Alkyl und (C₁-C₃)-Alkoxy ihrerseits durch Hydroxy oder Amino substituiert sein können,
und
R³ für Wasserstoff oder (C₁-C₆)-Alkyl, welches durch Hydroxy, (C₁-C₄)-Alkoxy, Amino oder Mono- oder Di-(C₁-C₄)-alkylamino substituiert sein kann, steht,
sowie ihre Salze, Solvate und Solvate der Salze.

2. Verbindung der Formel (I) nach Anspruch 1, in welcher
A für eine Gruppe der Formel 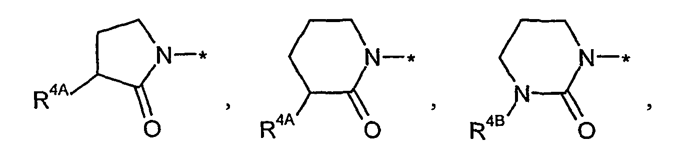 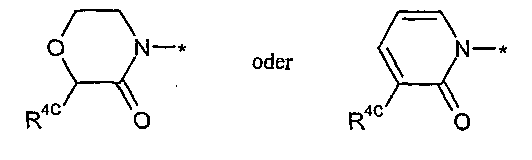 steht, worin
R^{4A} Wasserstoff, Hydroxy, Methoxy oder Amino,
R^{4B} Methyl oder Ethyl, welche jeweils durch Hydroxy, Amino, Pyrrolidino oder Cyclopropylamino substituiert sein können, oder Amino,
R^{4C} Wasserstoff, Methyl oder Ethyl, wobei Methyl oder Ethyl jeweils durch Hydroxy, Amino, Pyrrolidino oder Cyclopropylamino substituiert sein können,
und
* die Verknüpfungsstelle mit dem Phenylring bedeuten,
Z für eine Gruppe der Formel 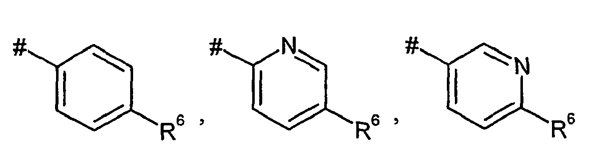 oder 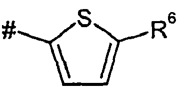 steht, worin
R⁶ Fluor, Chlor, Methyl, Cyano oder Ethinyl
und
# die Verknüpfungsstelle mit dem Stickstoffatom bedeutet,
R¹ für Wasserstoff,
R² für Wasserstoff, Fluor oder Methyl,
und
R³ für Wasserstoff stehen,
sowie ihre Salze, Solvate und Solvate der Salze.

3. Verbindung der Formel (I) nach Anspruch 1 oder 2, in welcher
A für eine heterocyclische Gruppe der Formel 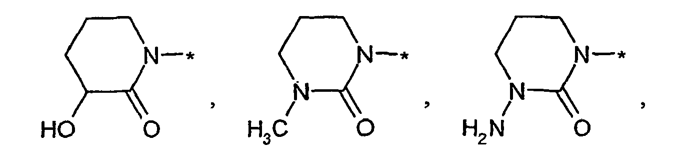 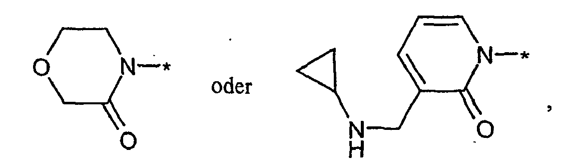 worin * die Verknüpfungsstelle mit dem Phenylring bedeutet,
Z für eine Gruppe der Formel 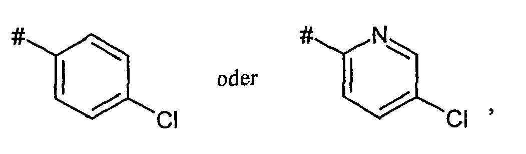 worin # die Verknüpfungsstelle mit dem Stickstoffatom bedeutet,
R¹ für Wasserstoff,
R² für Wasserstoff, Fluor oder Methyl,
und
R³ für Wasserstoff stehen,
sowie ihre Salze, Solvate und Solvate der Salze.

4. Verfahren zur Herstellung von Verbindungen der Formel (I), wie in Anspruch 1 definiert, **dadurch gekennzeichnet, dass** man entweder
[A] Verbindungen der Formel (II) 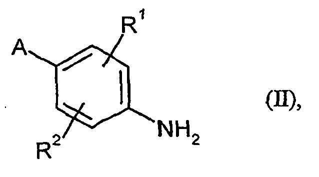 in welcher A, R¹ und R² die in Anspruch 1 angegebenen Bedeutungen haben, zunächst mit einer Verbindung der Formel (III) 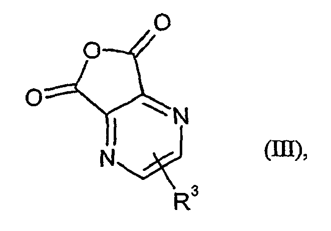 in welcher R³ die in Anspruch 1 angegebene Bedeutung hat, zu Verbindungen der Formel (IV) 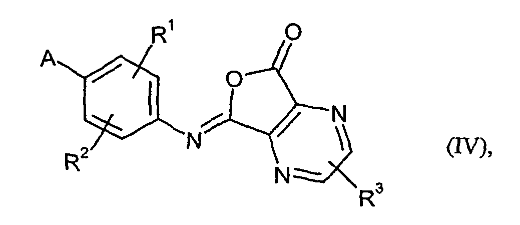 in welcher A, R¹, R² und R³ die in Anspruch 1 angegebenen Bedeutungen haben, umsetzt und diese dann mit einer Verbindung der Formel (V)
H₂N—Z (V),
in welcher Z die in Anspruch 1 angegebene Bedeutung hat,
in Verbindungen der Formel (I) überführt
oder
[B] Verbindungen der Formel (V) zunächst mit einer Verbindung der Formel (III) zu Verbindungen der Formel (VI) 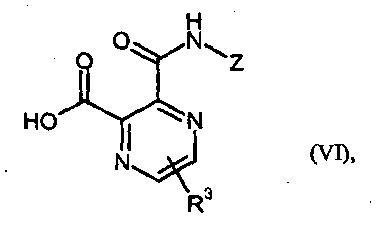 in welcher R³ und Z die in Anspruch 1 angegebenen Bedeutungen haben,
umsetzt und diese dann mit einer Verbindung der Formel (II) in Verbindungen der Formel (I) überführt,
und die Verbindungen der Formel (I) gegebenenfalls mit den entsprechenden (i) Lösungsmitteln und/oder (ii) Basen oder Säuren zu ihren Solvaten, Salzen und/oder Solvaten der Salze umsetzt.

5. Verbindung der Formel (I) sowie ihre physiologisch unbedenklichen Salze, Hydrate und Hydrate der physiologisch unbedenklichen Salze; wie in Anspruch 1 definiert, zur Behandlung und/oder Prophylaxe von Krankheiten.

6. Verwendung einer Verbindung der Formel (I) oder eines ihrer physiologisch unbedenklichen Salze, Hydrate oder Hydrate der physiologisch unbedenklichen Salze, wie in Anspruch 1 definiert, zur Herstellung eines Arzneimittels zur Behandlung und/oder Prophylaxe von thromboembolischen Erkrankungen.

7. Verwendung einer Verbindung der Formel (I) oder eines ihrer physiologisch unbedenklichen Salze, Hydrate oder Hydrate der physiologisch unbedenklichen Salze, wie in Anspruch 1 definiert, zur Verhinderung der Blutkoagulation in vitro.

8. Arzneimittel enthaltend eine Verbindung der Formel (I) oder eines ihrer physiologisch unbedenklichen Salze, Hydrate oder Hydrate der physiologisch unbedenklichen Salze, wie in Anspruch 1 definiert, in Kombination mit einem inerten, nicht-toxischen, pharmazeutisch geeigneten Hilfsstoff.

9. Arzneimittel enthaltend eine Verbindung der Formel (I) oder eines ihrer physiologisch unbedenklichen Salze, Hydrate oder Hydrate der physiologisch unbedenklichen Salze, wie in Anspruch 1 definiert, in Kombination mit einem weiteren Wirkstoff.

10. Arzneimittel nach Anspruch 8 oder 9 zur Behandlung und/oder Prophylaxe von thromboembolischen Erkrankungen.

11. Verfahren zur Verhinderung der Blutkoagulation in vitro, **dadurch gekennzeichnet, dass** eine antikoagulatorisch wirksame Menge einer Verbindung der Formel (I) oder eines ihrer physiologisch unbedenklichen Salze, Hydrate oder Hydrate der physiologisch unbedenklichen Salze, wie in Anspruch 1 definiert, zugegeben wird.

## Claims

1. Compound of the formula (I) 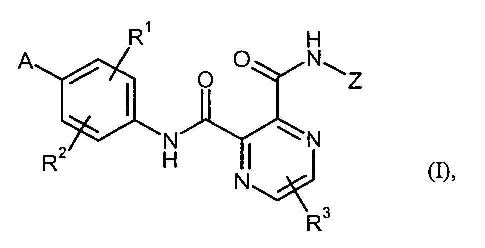 in which
A represents a group of the formula 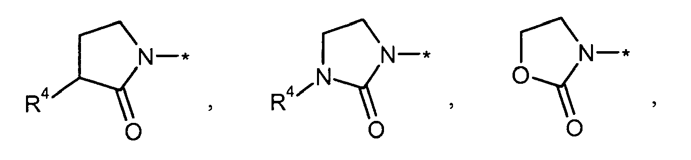 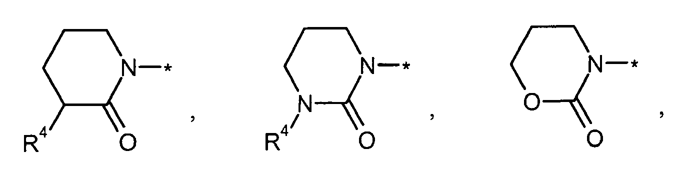 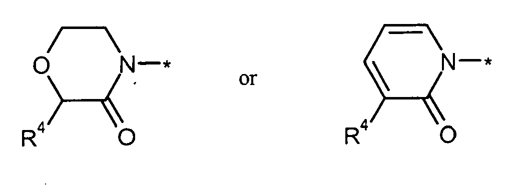 in which
R⁴ represents hydrogen, (C₁-C₆)-alkyl, hydroxyl, (C₁-C₆)-alkoxy, amino, mono- or di-(C₁-C₆)-alkylamino, (C₃-C₇)-cycloalkylamino, (C₁-C₆)-alkanoylamino or (C₁-C₆)-alkoxycarbonylamino, where
(C₁-C₆)-alkyl, (C₁-C₆)-alkoxy, mono- and di-(C₁-C₆)-alkylamino for their part may in each case be substituted by hydroxyl, (C₁-C₄)-alkoxy, amino, mono- or di-(C₁-C₄)-alkylamino, (C₃-C₇)-cycloalkylamino or a 4- to 7-membered saturated heterocycle which is attached via a nitrogen atom and which may contain a ring member from the group consisting of N-R⁵ and O, in which
R⁵ represents hydrogen or (C₁-C₄)-alkyl,
and * represents the point of attachment to the phenyl ring,
Z represents phenyl, pyridyl, pyrimidinyl, pyrazinyl or thienyl which may in each case be mono- or disubstituted by identical or different substituents selected from the group consisting of fluorine, chlorine, cyano, (C₁-C₄)-alkyl (which for its part may be substituted by amino), ethynyl and amino,
R¹ and R² are identical or different and independently of one another represent hydrogen, fluorine, chlorine, cyano, (C₁-C₃)-alkyl, cyclopropyl, trifluoromethyl, hydroxyl, (C₁-C₃)-alkoxy, trifluoromethoxy or amino, where
(C₁-C₃)-alkyl and (C₁-C₃)-alkoxy for their part may be substituted by hydroxyl or amino,
and
R³ represents hydrogen or (C₁-C₆)-alkyl, which may be substituted by hydroxyl, (C₁-C₄)-alkoxy, amino or mono- or di-(C₁-C₄)-alkylamino,
and salts, solvates and solvates of the salts thereof.

2. Compound of the formula (I) according to Claim 1 in which
A represents a group of the formula 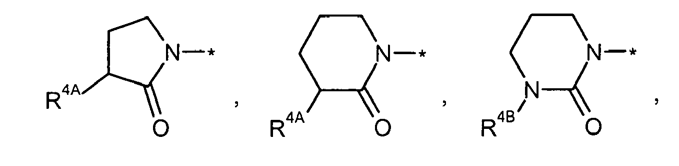 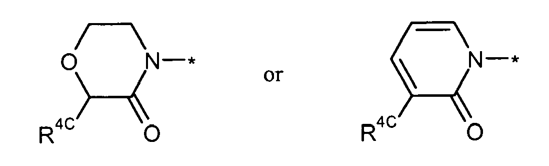 in which
R^{4A} represents hydrogen, hydroxyl, methoxy or amino,
R^{4B} represents methyl or ethyl, each of which may be substituted by hydroxyl, amino, pyrrolidino or cyclopropylamino, or amino,
R^{4C} represents hydrogen, methyl or ethyl, where methyl or ethyl may in each case be substituted by hydroxyl, amino, pyrrolidino or cyclopropylamino,
and
* represents the point of attachment to the phenyl ring,
Z represents a group of the formula 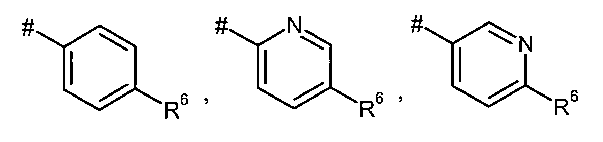 or 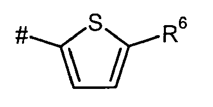 in which
R⁶ represents fluorine, chlorine, methyl, cyano or ethynyl
and
# represents the point of attachment to the nitrogen atom,
R¹ represents hydrogen,
R² represents hydrogen, fluorine or methyl,
and
R³ represents hydrogen,
and salts, solvates and solvates of the salts thereof.

3. Compound of the formula (I) according to Claim 1 or 2 in which
A represents a heterocyclic group of the formula 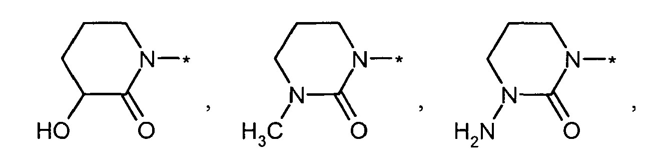 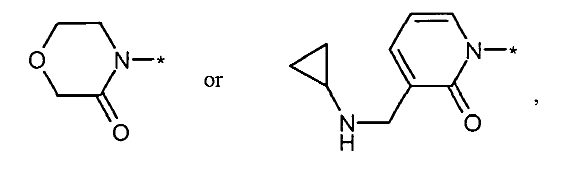 in which * represents the point of attachment to the phenyl ring,
Z represents a group of the formula 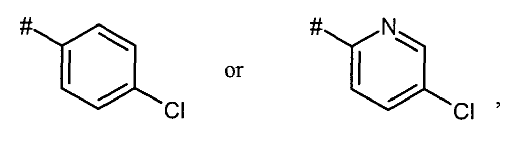 in which # represents the point of attachment to the nitrogen atom,
R¹ represents hydrogen,
R² represents hydrogen, fluorine or methyl,
and
R³ represents hydrogen,
and salts, solvates and solvates of the salts thereof.

4. Process for preparing compounds of the formula (I) as defined in Claim 1, **characterized in that** either
[A] compounds of the formula (II) 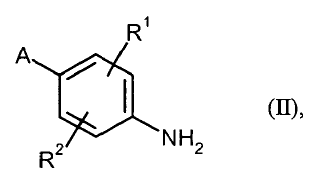 in which A, R¹ and R² are as defined in Claim 1 are initially reacted with a compound of the formula (III) 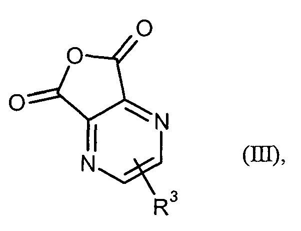 in which R³ is as defined in Claim 1 to give compounds of the formula (IV) 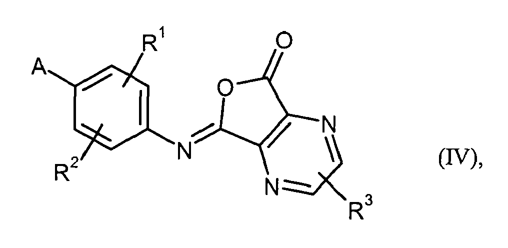 in which A, R¹, R² and R³ are as defined in Claim 1
and these are then converted with a compound of the formula (V)
H₂N—Z (V),
in which Z is as defined in Claim 1
into compounds of the formula (I)
or
[B] compounds of the formula (V) are initially reacted with a compound of the formula (III) to give compounds of the formula (VI) 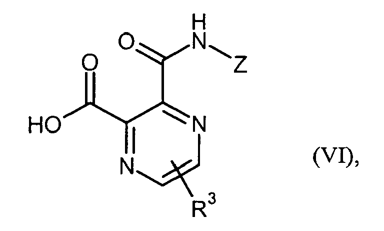 in which R³ and Z are as defined in Claim 1
and these are then converted with a compound of the formula (II) into compounds of the formula (I),
and the compounds of the formula (I) are, if appropriate, converted with the appropriate (i) solvents and/or (ii) bases or acids into their solvates, salts and/or solvates of the salts.

5. Compound of the formula (I) and physiologically acceptable salts, hydrates and hydrates of the physiologically acceptable salts thereof as defined in Claim 1 for the treatment and/or prophylaxis of diseases.

6. Use of a compound of the formula (I) or one of the physiologically acceptable salts, hydrates or hydrates of the physiologically acceptable salts thereof as defined in Claim 1 for preparing a medicament for the treatment and/or prophylaxis of thromboembolic disorders.

7. Use of a compound of the formula (I) or one of the physiologically acceptable salts, hydrates or hydrates of the physiologically acceptable salts thereof as defined in Claim 1 for preventing blood coagulation in vitro.

8. Medicament, comprising a compound of the formula (I) or one of the physiologically acceptable salts, hydrates or hydrates of the physiologically acceptable salts thereof as defined in Claim 1 in combination with an inert nontoxic pharmaceutically acceptable auxiliary.

9. Medicament, comprising a compound of the formula (I) or one of the physiologically acceptable salts, hydrates or hydrates of the physiologically acceptable salts thereof as defined in Claim 1 in combination with a further active compound.

10. Medicament according to Claim 8 or 9 for the treatment and/or prophylaxis of thromboembolic disorders.

11. Method for prevening blood coagulation in vitro, **characterized in that** an anticoagulatory effective amount of a compound of the formula (I) or one of the physiologically acceptable salts, hydrates or hydrates of the physiologically acceptable salts thereof as defined in Claim 1 is added.

## Revendications

1. Composé de formule (I) 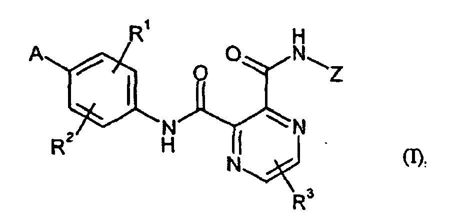 dans laquelle
A représente un groupe de formule 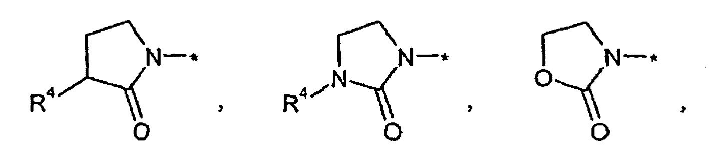 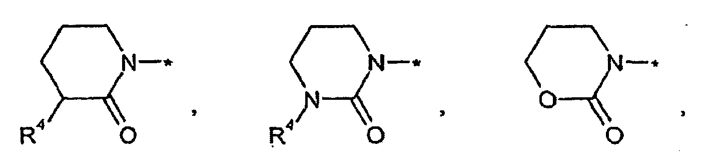 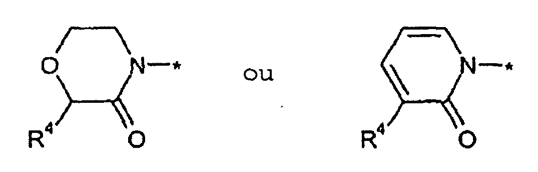 où
R⁴ représente l'hydrogène, un reste alkyle en C₁ à C₆, hydroxy, alkoxy en C₁ à C₆, amino, mono- ou di-(alkyle en C₁ à C₆) amino, (cycloalkyle en C₃ à C₇)-amino, (alcanoyle en C₁ à C₆) amino ou (alkoxy en C₁ à C₆) carbonylamino,
les restes alkyle en C₁ à C₆, alkoxy en C₁ à C₆, mono- et di-(alkyle en C₁ à C₆) amino pouvant eux-mêmes être substitués dans chaque cas par un radical hydroxy, alkoxy en C₁ à C₄, amino, mono- ou di-(alkyle en C₁ à C₄) amino, (cycloalkyle en C₃ à C₇) amino ou par un hétérocycle tétragonal à heptagonal saturé, lié par l'intermédiaire d'un atome d'azote et pouvant contenir dans son noyau un chaînon de la série N-R⁵ ou O, où
R⁵ représente l'hydrogène ou un reste alkyle en C₁ à C₄,
et le signe * désigne la position de rattachement au noyau phényle,
Z représente un reste phényle, pyridyle, pyrimidinyle, pyrazinyle ou thiényle dont chacun peut être substitué une ou plusieurs fois identiques ou différentes par des substituants choisis dans le groupe fluor, chlore, cyano, alkyle en C₁ à C₄ qui peut lui-même être substitué par un radical amino,
R¹ et R² sont identiques ou différents et représentent, indépendamment l'un de l'autre, l'hydrogène, le fluor, le chlore, un reste cyano, alkyle en C₁ à C₃, cyclopropyle, trifluorométhyle, hydroxy, alkoxy en C₁ à C₃, trifluorométhoxy ou amino,
les restes alkyle en C₁ à C₃ et alkoxy en C₁ à C₃ pouvant eux-mêmes être substitués par un radical hydroxy ou amino,
et
R³ représente l'hydrogène ou un reste alkyle en C₁ à C₆, qui peut être substitué par un radical hydroxy, alkoxy en C₁ à C₄, amino ou mono- ou di-(alkyle en C₁ à C₄) - amino,
ainsi que ses sels, ses produits de solvatation et les produits de solvatation des sels.

2. Composé de formule (I) suivant la revendication 1, formule dans laquelle
A représente un groupe de formule 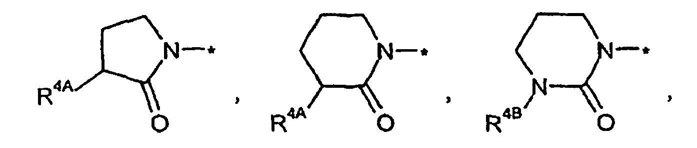 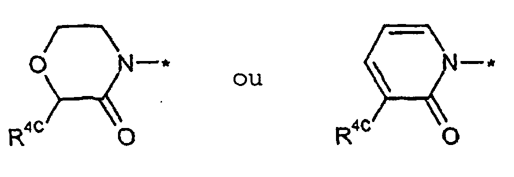 où
R^{4A} représente l'hydrogène, un reste hydroxy, méthoxy ou amino,
R^{4B} représente un reste méthyle ou un reste éthyle, dont chacun peut être substitué par un radical hydroxy, amino, pyrrolidino ou cyclopropylamino, ou bien un reste amino,
R^{4C} représente l'hydrogène, un reste méthyle ou un reste éthyle, chaque reste méthyle ou éthyle pouvant être substitué par un radical hydroxy, amino, pyrrolidino ou cyclopropylamino,
et
* désigne la position de rattachement au noyau phényle,
Z représente un groupe de formule 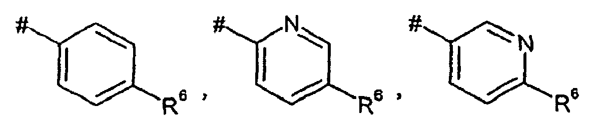 ou 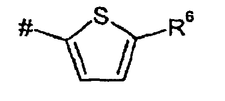 où
R⁶ représente le fluor, le chlore, un reste méthyle, cyano ou éthynyle,
et
# représente la position de rattachement à l'atome d'azote,
R¹ représente l'hydrogène,
R² représente l'hydrogène, le fluor ou un groupe méthyle,
et
R³ représente l'hydrogène,
ainsi que ses sels, ses produits de solvatation et les produits de solvatation des sels.

3. Composé de formule (I) suivant la revendication 1 ou 2, formule dans laquelle
A représente un groupe hétérocyclique de formule 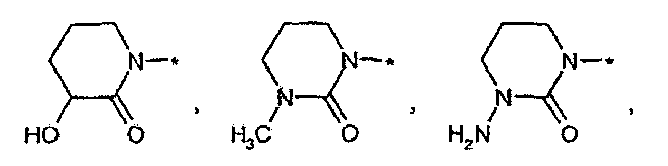 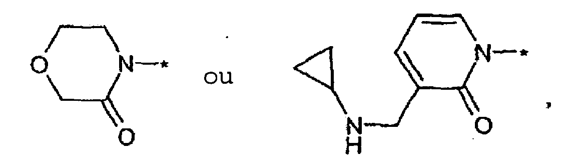 où * désigne la position de rattachement au noyau phényle,
Z représente un groupe de formule 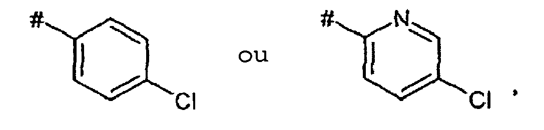 où le signe # indique la position de rattachement à l'atome d'azote,
R¹ représente l'hydrogène,
R² représente l'hydrogène, le fluor ou un reste méthyle,
et
R³ représente l'hydrogène,
ainsi que ses sels, ses produits de solvatation et les produits de solvatation des sels.

4. Procédé de production de composés de formule (I), telle que définie dans la revendication 1, **caractérisé en ce que**
[A] des composés de formule (II) 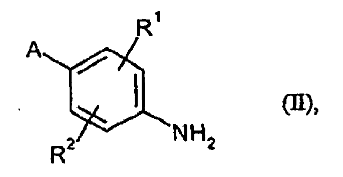 dans laquelle A, R¹ et R² ont les définitions indiquées dans la revendication 1,
sont tout d'abord amenés à réagir avec un composé de formule (III) 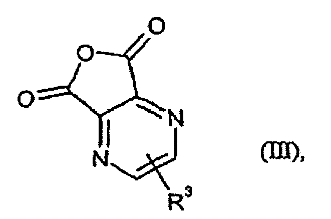 dans laquelle R³ a la définition indiquée dans la revendication 1,
pour former des composés de formule (IV) 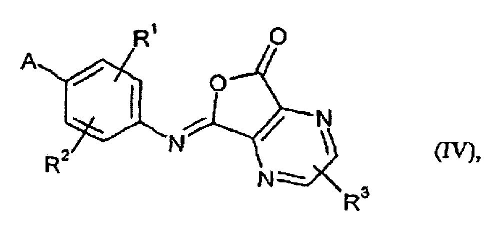 dans laquelle A, R¹, R² et R³ ont les définitions indiquées dans la revendication 1,
et ces composés sont ensuite transformés avec un composé de formule (V)
H₂N—Z (V),
dans laquelle Z a la définition indiquée dans la revendication 1,
en composés de formule (I)
ou bien
[B] des composés de formule (V) sont tout d'abord amenés à réagir avec un composé de formule (III) pour produire des composés de formule (VI) 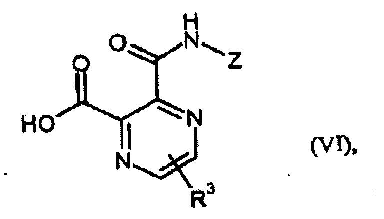 dans laquelle R³ et Z ont les définitions indiquées dans la revendication 1,
et ces composés sont ensuite transformés avec un composé de formule (II) en composés de formule (I),
et des composés de formule (I) sont éventuellement transformés avec (i) les solvants correspondants et/ou (ii) les bases ou les acides correspondants en leurs produits de solvatation, leurs sels et/ou les produits de solvatation des sels.

5. Composé de formule (I) ainsi que ses sels physiologiquement acceptables, ses hydrates et les hydrates des sels physiologiquement acceptables, comme défini dans la revendication 1, destiné au traitement et/ou à la prophylaxie de maladies.

6. Utilisation d'un composé de formule (I) ou de l'un de ses sels physiologiquement acceptables, de ses hydrates ou des hydrates des sels physiologiquement acceptables, comme défini dans la revendication 1, pour la préparation d'un médicament destiné au traitement et/ou à la prophylaxie de maladies thromboemboliques.

7. Utilisation d'un composé de formule (I) ou de l'un de ses sels physiologiquement acceptables, de ses hydrates ou des hydrates des sels physiologiquement acceptables, comme défini dans la revendication 1, pour empêcher la coagulation de sang in vitro.

8. Médicament contenant un composé de formule (I) ou l'un de ses sels physiologiquement acceptables, de ses hydrates ou des hydrates des sels physiologiquement acceptables, comme défini dans la revendication 1, en combinaison avec une substance auxiliaire inerte non toxique pharmaceutiquement acceptable.

9. Médicament contenant un composé de formule (I) ou l'un de ses sels physiologiquement acceptables, de ses hydrates ou des hydrates des sels physiologiquement acceptables, comme défini dans la revendication 1, en combinaison avec une autre substance active.

10. Médicament suivant la revendication 8 ou 9, destiné au traitement et/ou à la prophylaxie de maladies thromboemboliques.

11. Procédé destiné à empêcher la coagulation de sang in vitro, **caractérisé en ce qu'**on ajoute une quantité à action anticoagulante d'un composé de formule (I) ou d'un de ses sels physiologiquement acceptables, de ses hydrates ou des hydrates des sels physiologiquement acceptables, comme défini dans la revendication 1.
